# EUROPEAN PATENT APPLICATION

(11) **EP 2 294 912 A1**
(43) Date of publication of application: **16.03.2011**
(21) Application number: 09169539.5
(22) Date of filing: 04.09.2009
(51) Int. Cl.: A01H 1/04, A01H 5/04, C12Q 1/68, A23L 1/052

(54) **Method for increasing the level of zeaxanthin in a plant line, method for selecting a plant or part thereof, including a seed and tuber, and use thereof**

(71) Applicant: Wageningen Universiteit, 6701 BH Wageningen (NL); Stichting voor de Technische Wetenschappen, 3527 JP Utrecht (NL)
(72) Inventor: The designation of the inventor has not yet been filed
(74) Representative: Hatzmann, Martin

(57) **Abstract**

The present invention relates to methods for increasing the level of zeaxanthin in a plant line. The invention also relates to such methods for selecting a plant or part thereof, including a seed and tuber, comprising a B-carotene hydroxylase (CHY2) gene. The invention further relates to the use of a marker for selecting a plant or part thereof, including a seed, with a genotype comprising at least one recessive zeaxanthin epoxidase (ZEP) allele. The invention even further relates to the use of a method for selecting a plant or part thereof including a seed, comprising a dominant CHY2 allele. The invention further relates to the use of a marker for detecting the required ZEP allele(s) in a plant or part thereof, and the use of such a marker for marker-assisted selection of plants. The invention also relates to a tetraploid potato having deep-yellow or orange tuber flesh.

## Description

### INTRODUCTION

The present invention relates to methods for increasing the level of zeaxanthin in a plant line. The invention also relates to such methods for selecting a plant or part thereof, including a seed and tuber, comprising a β-carotene hydroxylase (CHY2) gene. The invention further relates to the use of a marker for selecting a plant or part thereof, including a seed, with a genotype comprising at least one recessive zeaxanthin epoxidase (ZEP) allele. The invention even further relates to the use of a method for selecting a plant or part thereof including a seed, comprising a dominant CHY2 allele. The invention further relates to the use of a marker for detecting the required ZEP allele(s) in a plant or part thereof, and the use of such a marker for marker-assisted selection of plants. The invention also relates to a tetraploid potato having orange tuber flesh.

### BACKGROUND OF THE INVENTION

Potato is a healthy and nutritious part of the average Western human diet, contributing carbohydrates and important amino acids and vitamins. Potato is available in cultivars with white, cream and yellow tuber flesh. This yellow colour is predominantly caused by the presence of carotenoids. A small number of cultivars have red or blue/purple flesh, caused by the presence of anthocyanins.

The main carotenoids present in cultivated potato are lutein, violaxanthin, zeaxanthin, and antheraxanthin (Breithaupt and Bamedi 2002; Brown *et al.* 1993; Iwanzik *et al.* 1983; Nesterenko and Sink, 2003). Beta-carotene, the precursor of vitamin A, is almost absent in *S. tuberosum* genotypes or closely related *Solanum* species (Breithaupt and Bamedi 2002). Carotenoids are recognized as important health promoting ingredients of the human diet. Some have antioxidant properties, and are supposedly beneficial in preventing cancer, cardiac disease, and eye diseases (Krinsky *et al.* 2004). Lutein and zeaxanthin are thought to be important in the human diet to prevent age-related macular eye degeneration (AMD) (Moeller *et al.* 2006; Seddon *et al.* 1994; Snodderly 1995). Lutein and zeaxanthin are components of the *macula lutea* in the human eye (Handelman *et al.* 1988), protecting the retina against damaging irradiation. Lutein and zeaxanthin have to be replenished constantly. As humans can not produce lutein and zeaxanthin themselves these antioxidants have to be consumed by eating carotenoid-rich plant products. Lutein is present in high amounts in dark green leafy vegetables such as spinach and kale. Zeaxanthin however is less abundant in most vegetables (Sommerburg *et al.* 1998). Thus, it would be beneficial to human health to increase the level of zeaxanthin in plants.

In potato, lutein is present in relatively large amounts, whereas zeaxanthin is present in lower amounts (Breithaupt and Bamedi 2002; Nesterenko and Sink, 2003). However, some *Solanum* species closely related to *S. tuberosum* produce deep yellow or orange-fleshed tubers, caused by high zeaxanthin content (Andre *et al.* 2007). These are known as 'Papa Amarilla' in the Andean region. They belong to the diploid Andean species *S. stenotomum, S. goniocalyx* and *S. phureja* (Brown *et al.* 2007; Burgos *et al.* 2009). Brown *et al.* (2007) and Brown (2008) observed a relationship between ploidy level and total carotenoid content in 38 native South American cultivars, but could not offer an explanation. Significantly higher mean levels of total carotenoids were observed in diploid *S. phureja* accessions when compared to tetraploid cultivars. For example, Morris *et al.* (2004) describe a diploid high carotenoid-accumulating *S. phureja* accession (DB375\1, or Inca Dawn) that predominantly contains zeaxanthin.

Unfortunately, this diploid genotype like most of the diploid potatoes has a lower yield than tetraploid *S. tuberosum* cultivars (Bradshaw and Ramsay, 2005). Also Kobayashi *et al.* (2008) bred a diploid potato genotype with orange tuber flesh and very high zeaxanthin content. This genotype was derived from *S. phureja.* Again, this diploid variety has a lower yield than conventional tetraploid cultivars.

Because of the difference in yield capacity between diploid potato genotypes and tetraploid potato cultivars, there is a need for a method to breed (high yielding) tetraploid potato cultivars that have an increased level of zeaxanthin. Such high-yielding orange-fleshed tetraploid potato cultivars currently do not exist in nature. Orange-fleshed potatoes may aid in the recommended daily uptake of zeaxanthin, as potatoes and potato products constitute a considerable part of the human diet in the Western world. Furthermore, an additional utility of orange-fleshed potatoes is their attractive colour, enabling the production of decorative potatoes, processed potato products and food servings.

Yellow flesh colour in potato is mainly dependent on the presence of a dominant allele (Fruwirth 1912) at the Y (Yellow) locus. The Ylocus has been mapped on chromosome 3 of potato by Bonierbale *et al.* (1988). The most likely candidate for the gene involved in yellow flesh colour is β-carotene hydroxylase (abbreviated to BCH or CHY2) (Brown *et al.* 2006). This gene has been mapped at the same position as the Y locus (Thorup *et al.* 2000). However, as far as we are aware compelling evidence, about the relation between the CHY2 gene effect and the yellow colour phenotype (via transgenic complementation of a white fleshed individual, or the silencing of the colour phenotype in a yellow fleshed individual) has not been published in literature.

In spite of many genetic studies during the previous century, the gene(s) responsible for the orange (or deep-yellow) tuber flesh colour have not been described. Even using the more simple genetic stock material provided by diploid *Solanum* species has not resulted in an understanding of the inheritance of this trait. Brown *et al.* (1993) observed progeny with orange flesh colour and high levels of zeaxanthin in a hybrid population of the diploid Andean species *S. phureja-S. stenotomum.* They suggested that the orange phenotype was caused by a dominant Or allele at or close to the Y locus on chromosome 3 of potato. However, allelism of Or as an allele of the Y locus was not corroborated by later research, as Brown (2008) reported. Apparently, thus, there was a lack of transmissability outside the immediate 'Papa Amarilla' gene pool. In cauliflower an Or gene was cloned, responsible for orange-coloured curds (Lopez *et al.* 2008). This gene was found not to be involved in the carotenoid biosynthetic pathway, but to control chromoplast differentiation, resulting in the sequestering of large amounts of carotenoids.

Orange tuber flesh in potatoes may be caused by changes in the activity of the enzyme zeaxanthin epoxidase (ZEP). ZEP is involved in the conversion of zeaxanthin into antheraxanthin, and in the conversion of antheraxanthin into violaxanthin (Tanaka *et al.* 2008). The level of zeaxanthin in a plant is inversely correlated with the level of activity of the plant's ZEP gene. For example, Morris et al (2004) observed an inversed trend between the level of ZEP transcript and tuber carotenoid content in a range of potato germplasm. Similarly, lower expression of the ZEP gene results in the accumulation of zeaxanthin, at the expense of antheraxanthin, violaxanthin and neoxanthin (Tanaka *et al.* 2008).

Methods for reducing such activity in a plant are known in the art. For example, WO 02/10302 and Römer *et al.* 2002 disclose a method for reducing the ZEP activity in a plant or plant cell, such as a potato. The method comprises the use of recombinant DNA technology to silence a plant's ZEP gene. The method provides plants such as tetraploid potatoes having increased zeaxanthin levels and increased total carotenoid contents. WO 2005/098005 discloses a similar recombinant method for reducing ZEP gene activity, involving the use of a special virus-based vector that is introduced in a target plant.

However, these transgenic methods are highly undesirable. First, transgenic methods are expensive due to high regulatory costs. Second, methods to produce transgenic crops are considered non-ethically by the environmentalists and lack consumer acceptance. Thus, a method for increasing the level of zeaxanthin in a plant is needed that does not involve the use of recombinant techniques and that does not provide transgenic plants. Recombinant DNA techniques resulting in the suppression of gene expression act irrespective of the ploidy level. Therefore, the method for increasing the level of zeaxanthin should enable the breeding at the tetraploid level. Preferably, such a method is cheap and involves biological methods such as crossing of a plant line.

Unfortunately, increasing the zeaxanthin level in a plant, in particular a diploid plant, by crossing alone is very difficult, but the feasibility of breeding for said traits at the tetraploid level is hitherto unknown and prohibitively complicated. For example, crossing orange diploid *S. phureja* genotypes with tetraploid *S. tuberosum* cultivars to obtain orange tetraploid progeny is complicated and time consuming as, despite over several decades of intensive research, present breeding techniques still apply classical methods for controlled pollination of parental clones. Hybrid seeds are sown in greenhouses and small seedling-tubers are harvested and retained from thousands of individual seedlings. The next year a single tuber from each resulting seedling is planted in the field, where extreme caution is exercised to avoid the spread of virus and diseases. From this first-year seedling crop, several "seed" tubers from each hybrid individual which survived the selection process are retained for the next year's planting.

After the second year, samples are tested to determine the suitability of the tubers for commercial usage. Plants which have survived the selection process to this point are then planted at an expanded volume the third year for a more comprehensive series of usage test. At the fourth-year stage of development, surviving selections are subjected to field trials in several countries to determine their adaptability to different growing conditions. Eventually, the varieties having superior qualities are transferred to other farms and the seed increased to commercial scale. Generally, by this time, around ten years of planting, harvesting and testing have been invested in attempting to develop the new and improved potato cultivars.

Such crossing or breeding techniques are especially time consuming when no method for observing the genetic composition of the potato clones which would be responsible for the desired phenotype are available. It is therefore close to impossible to achieve orange potato plants by crossing alone. Thus, a method for increasing a plant line's zeaxanthin level is needed that preferably involves marker assisted selection of suitable parental genotypes, which would allow to predict the Mendelian segregation ratios in the offspring, and thus to enhance the probability to obtain the desired phenotype, wherein the selection of the offspring genotypes of these crossing comprises marker-assisted selection.

As mentioned before, an increased level of zeaxanthin and/or orange tuber flesh, can be found in diploid potato species. Specifically the native Andean material related to the diploid species *S. phureja* may be used as starting material to introgress said trait into *S. tuberosum* Group Tuberosum (tetraploid) cultivars, requiring the invention of breeding strategies for said trait and requiring full identification of the required hereditary elements allowing the expression of said trait. Even if one would pursue this strategy of introgression breeding from *S. phureja* to tetraploid potato cultivars the method disclosed in this invention has to be an integral element of said strategy. The current absence of tetraploid potato cultivars with an increased level of zeaxanthin and/or orange flesh, for which there would be an important economic market, proves that this alternative method using diploid material is prohibitively complicated.

### SUMMARY OF THE INVENTION

The current invention provides such a method. Provided is a method for increasing the level of zeaxanthin in a plant, comprising:
a) selecting at least one parental genotype comprising at least one recessive zeaxanthin epoxidase (ZEP) allele,
b) crossing said plant(s),
c) selecting progeny from said crossing for a genotype comprising at least one recessive ZEP allele,
d) selfing and /or further crossing said progeny selected in step a),
e) selecting progeny resulting from the crossing in step d) for a genotype comprising at least one recessive ZEP allele,
f) optionally repeating said steps of selfing and/or crossing and selection of steps d) and e) to provide a plant having a diploid genotype that is homozygous or a tetraploid genotype that is quadruplex for said recessive ZEP allele,
wherein at least one selection as performed in steps a), c) or e) is performed by marker-assisted selection and wherein said method optionally comprises increasing the ploidy or the use of unreduced gametes of the parental genotype selected at step a) or the progeny of step c) to tetraploid level before conducting step e).

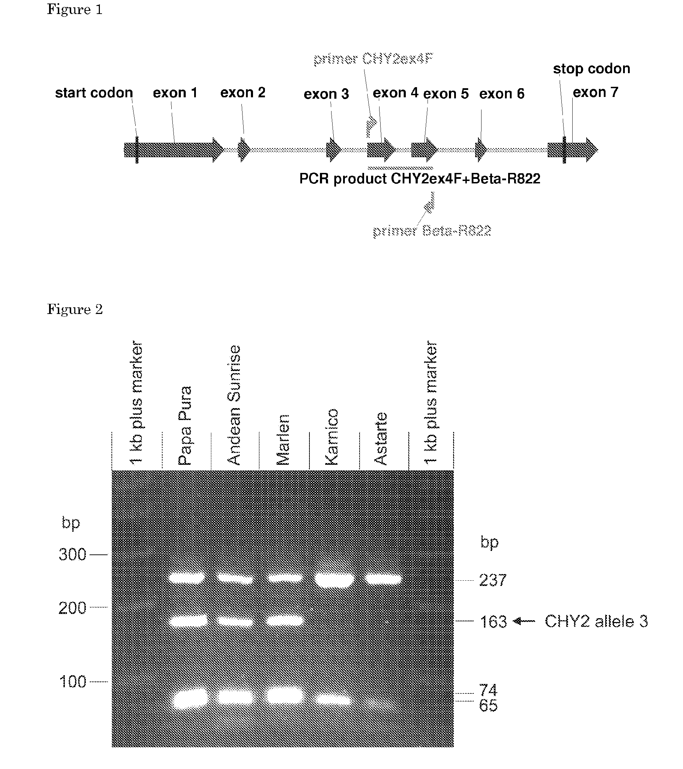

Preferably in said method the genotype of the plant in step f) or one or more of its parents comprises at least one dominant CHY2 allele, preferably wherein said CHY2 allele is indicated by a marker, for example the CAPS marker based on the PCR product generated with primers CHY2ex4F and Beta-R822 (Table 1, Figure 2), or any SNP selected from Table 2 detectable within PCR amplicon product CHY2ex4F and Beta-R822, , for example SNP T142C.

The invention further comprises a method for selecting a plant or part thereof, including a seed and tuber, said method comprising:
a) testing a plant or part thereof for the presence of at least one marker that is indicative for a recessive ZEP allele, and
b) selecting said plant or part thereof based on the information derived from said testing; and
c) optionally further testing a plant or part thereof for the presence of at least one marker that is indicative for a dominant CHY2 allele, and selecting said plant or part thereof based on the information derived from said testing.

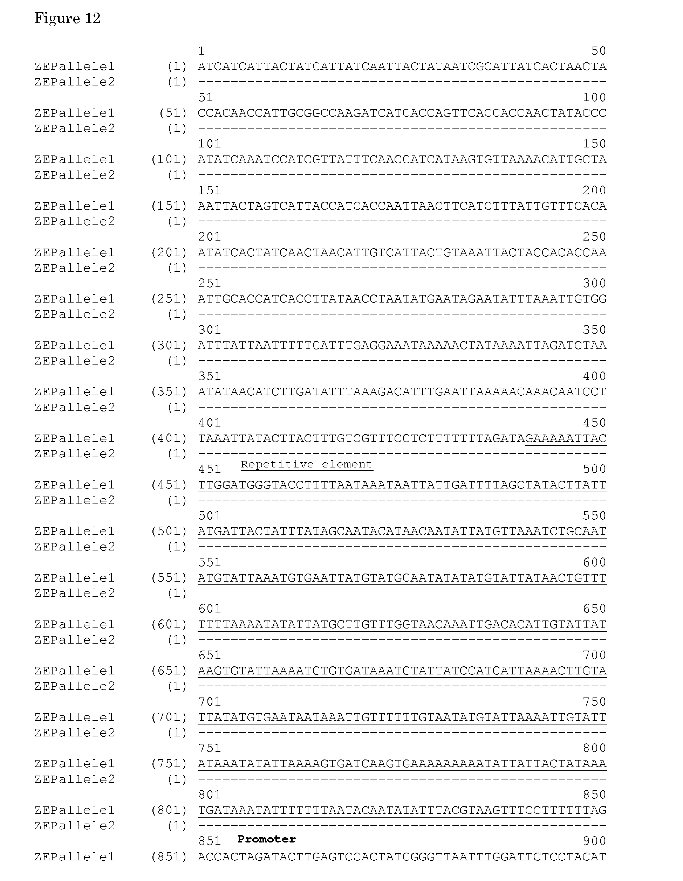

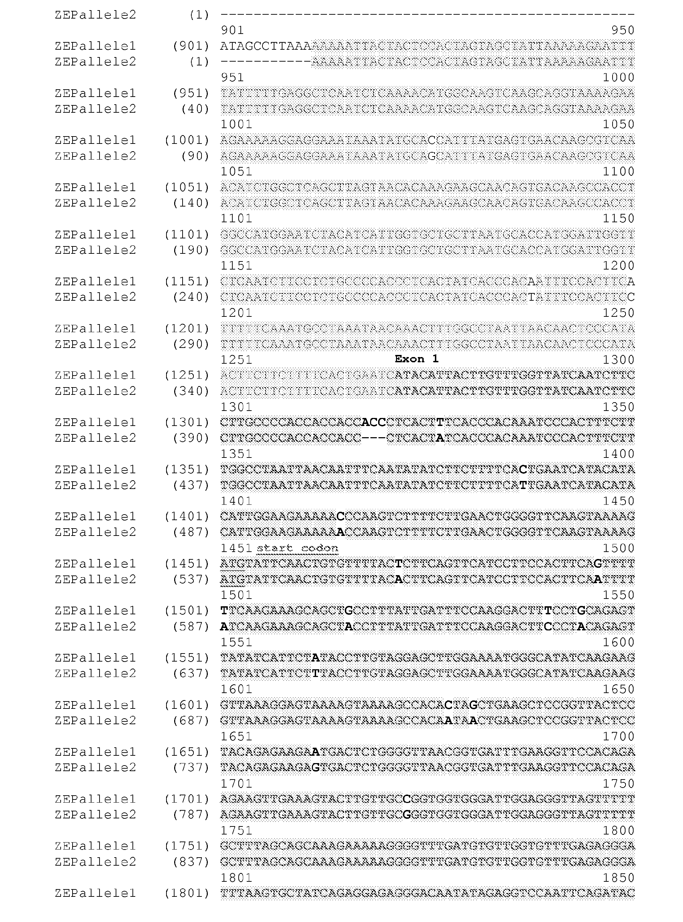

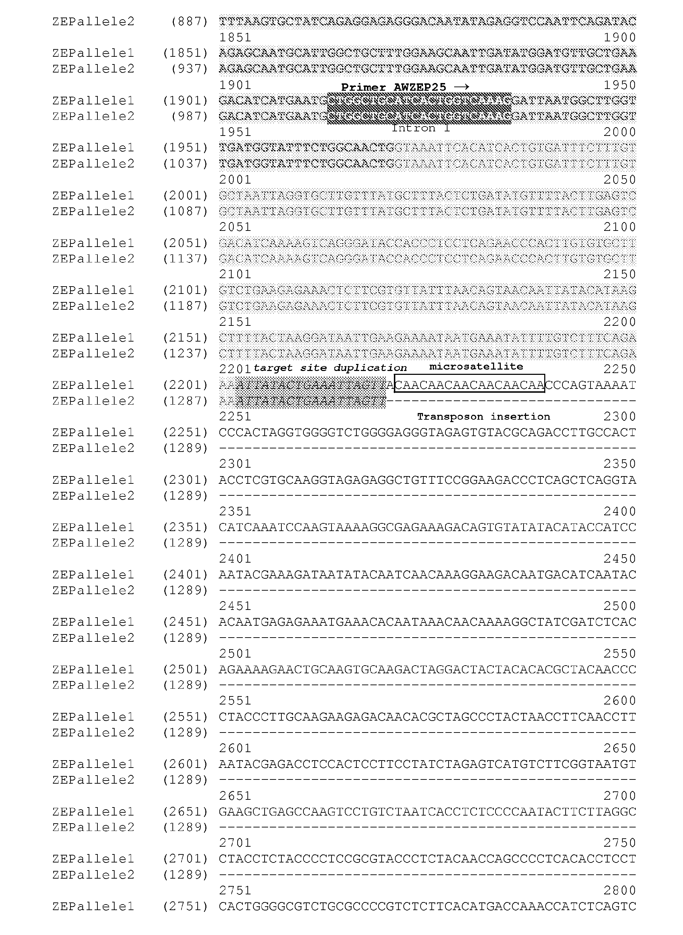

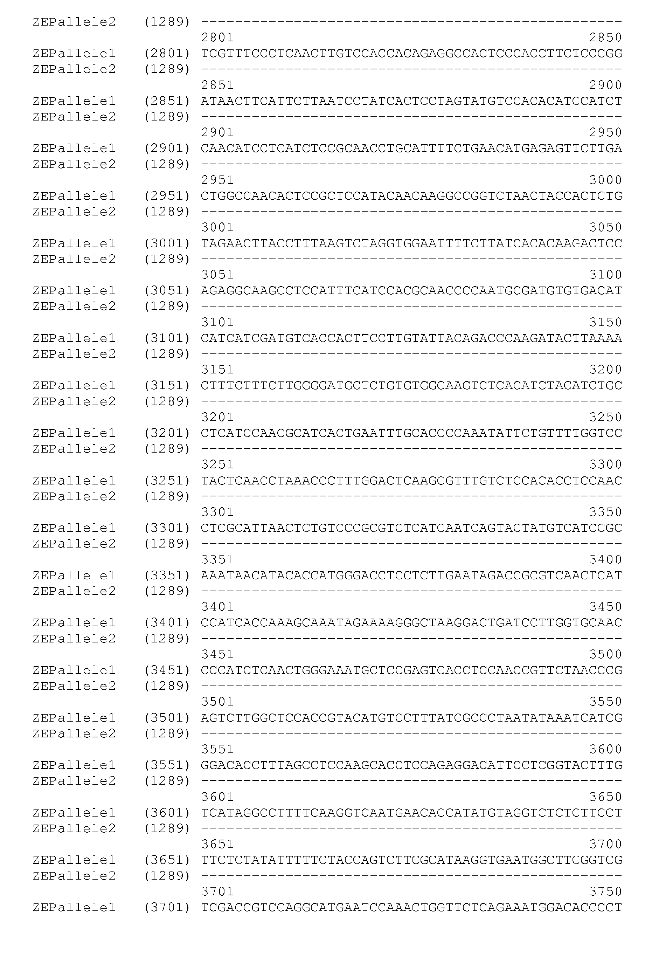

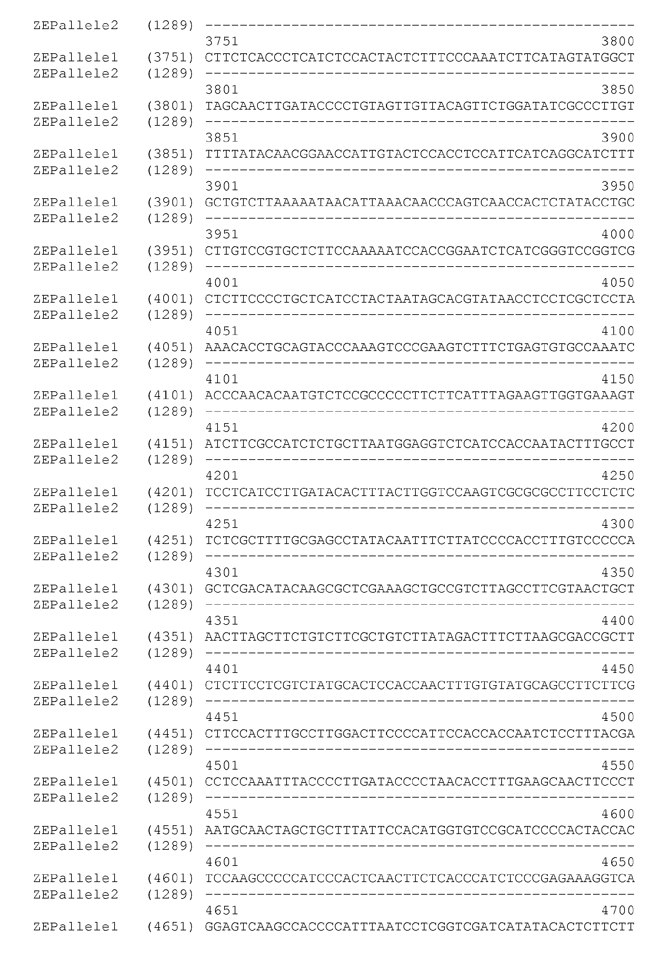

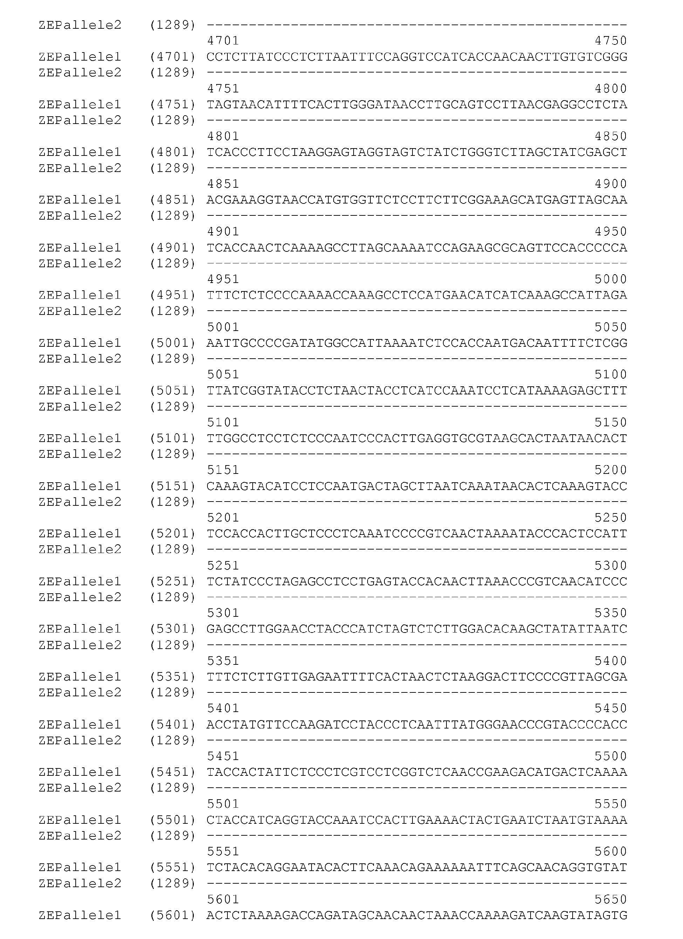

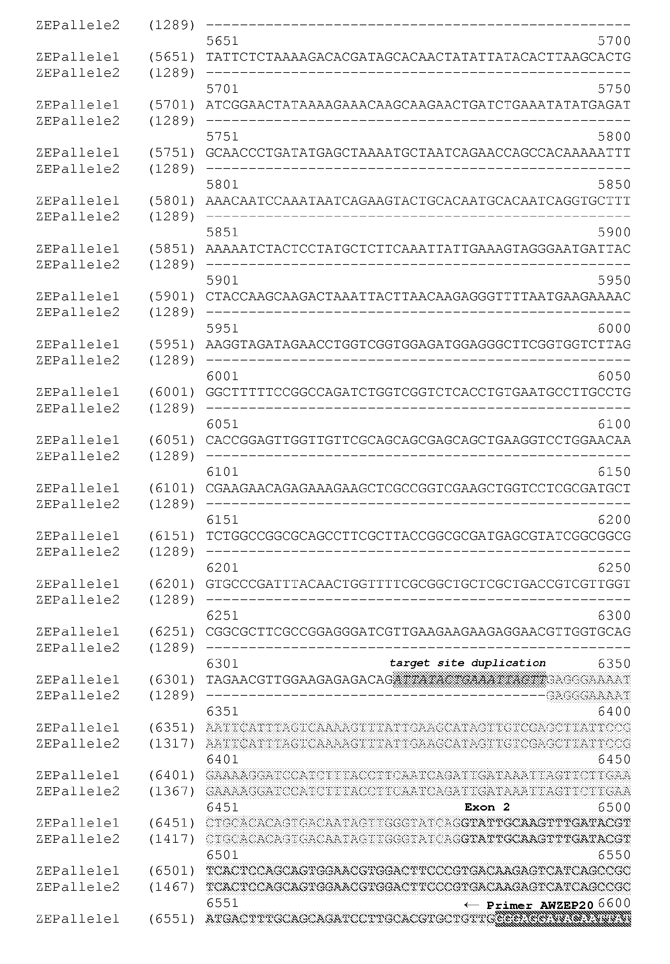

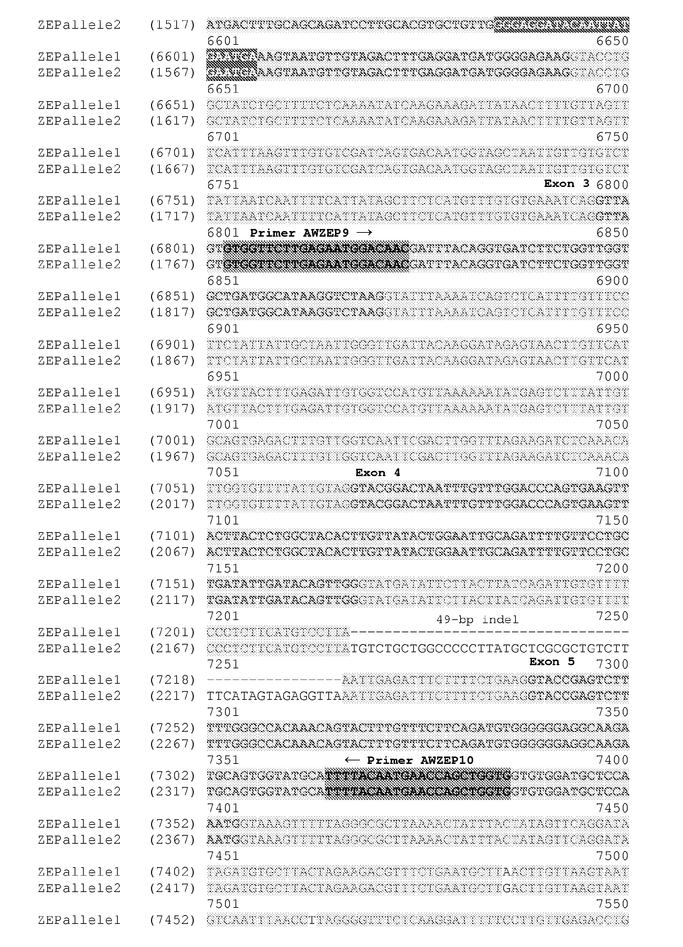

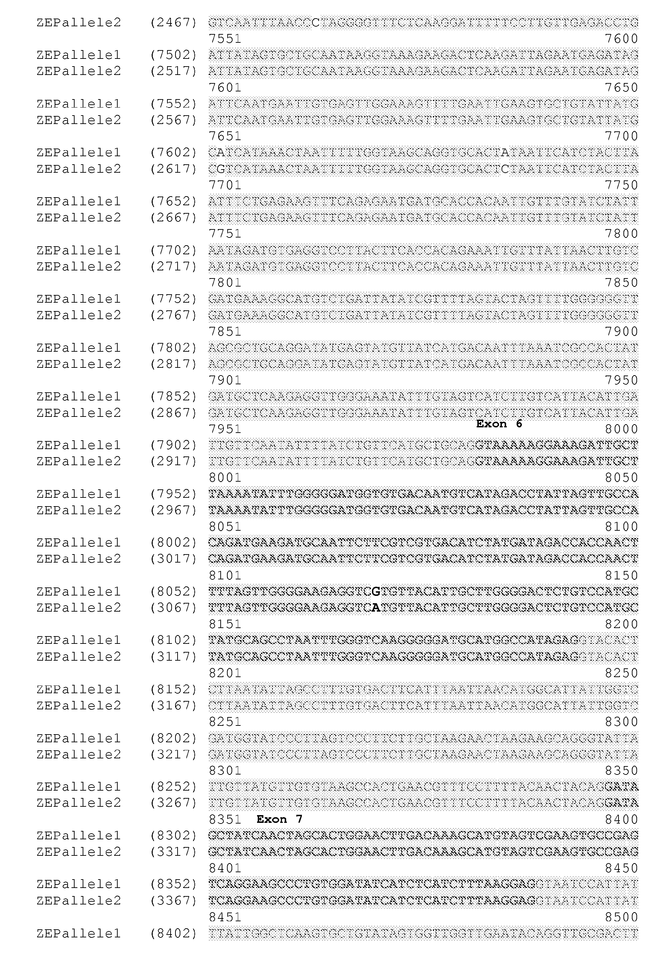

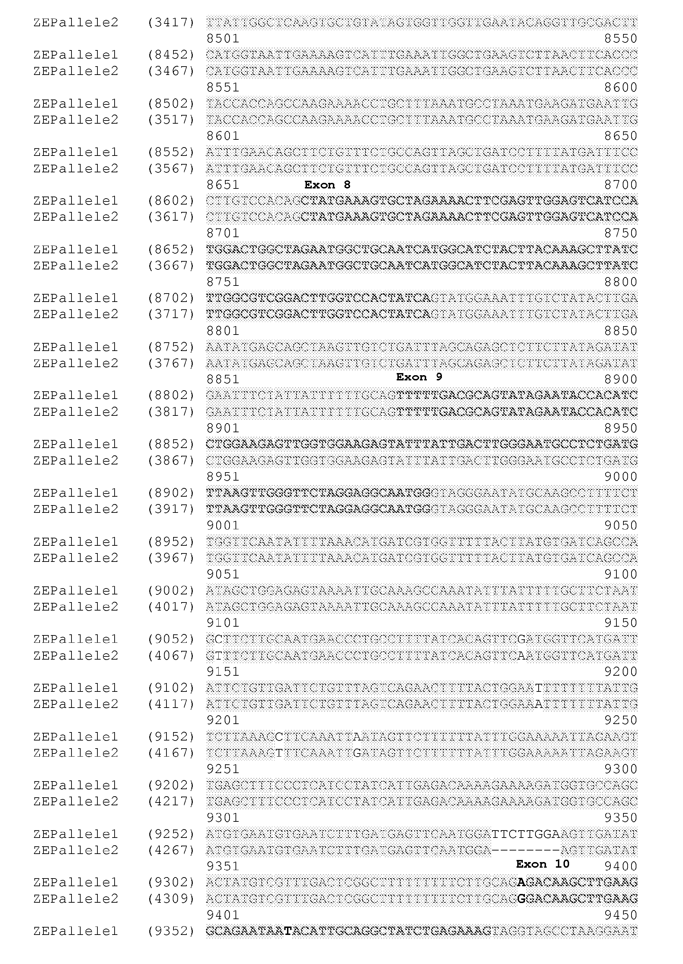

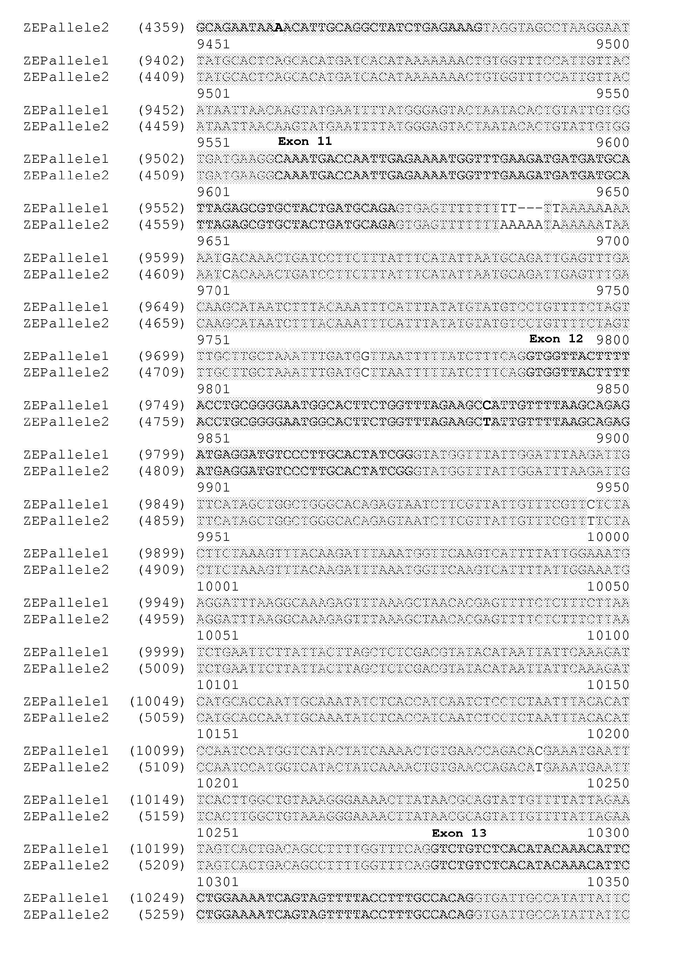

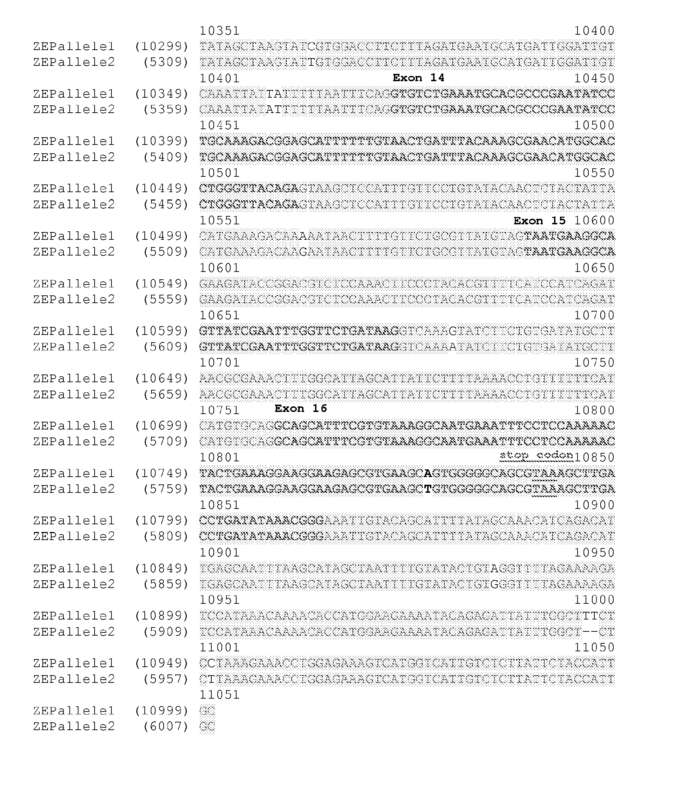

Preferably in a method according to the invention the recessive ZEP allele comprises the nucleic acid sequence of ZEP allele 1 in Figure 12. A further preferred embodiment is a method wherein the presence of said marker is determined at DNA level, preferably wherein said marker is selected from the group consisting of a single nucleotide polymorphism (SNP), an insertion and an indel, more preferably wherein said insertion is a transposon insertion, more preferably wherein said transposon insertion is the transposon insertion in intron 1 of the nucleic acid sequence of ZEP allele 1 in Figure 12. In another preferred embodiment the indel is the 49-base pairs indel in exon 4 of the nucleic acid sequence of ZEP allele 1 in Figure 12.

Preferably in a method according to the invention the plant is tetraploid. Moreover, it is preferred that the plant is a potato plant, preferably wherein a *S. tuberosum* Group Tuberosum cultivar.

The invention further comprises the use of a marker for selecting a plant or part thereof, including a seed and tuber, with a genotype comprising at least one recessive ZEP allele, wherein said marker is a SNP selected from the group consisting of G83A, T99G, A113T, A154G, G165C, A180G, T209C, T231A, T375G, A384T, A389C, A415G, C422-, C422T, T426- and T426C, the transposon insertion in intron 1 of the nucleic acid sequence of ZEP allele 1 in Figure 12 or the 49-base pairs indel in exon 4 of the nucleic acid sequence of ZEP allele 1 in Figure 12. In such a use the ZEP allele preferably comprises the nucleic acid sequence of ZEP allele 1 in Figure 12.

Further part of the invention is the use of a method according to the invention for marker-assisted crossing of a plant.

In a preferred embodiment the use according to the invention is directed to a tetraploid plant. In such an embodiment, preferably said plant is a potato plant, more preferably said potato plant is a *S. tuberosum* Group Tuberosum cultivar.

Also part of the invention is a probe for detecting a marker in a plant or part thereof, including a seed and tuber, wherein said marker is indicative for a ZEP allele comprising the nucleic acid sequence of ZEP allele 1 in Figure 12, preferably wherein said marker is a SNP selected from the group consisting of G83A, T99G, A113T, A154G, G165C, A180G, T209C, T231A, T375G, A384T, A389C, A415G, C422-, C422T, T426- and T426C, the transposon insertion in intron 1 of the nucleic acid sequence of ZEP allele 1 in Figure 12 or the 49-base pairs indel in exon 4 of the nucleic acid sequence of ZEP allele 1 in Figure 12.

In a further embodiment such a probe is used for marker-assisted breeding of a plant, preferably wherein said plant is tetraploid, more preferably wherein said plant is a potato plant, even more preferably wherein said potato plant is a *S. tuberosum* Group *tuberosum* cultivar.

Further, the invention comprises a tetraploid potato plant having tubers with an increased zeaxanthin content, wherein the genotype of said potato comprises homozygous recessive ZEP alleles comprising the nucleic acid sequence of ZEP allele 1 in Figure 12, preferably wherein said plant comprises at least one dominant CHY2 allele. Preferably said potato plant is a *S. tuberosum* Group Tuberosum cultivar, more preferably said potato plant is a tetraploid *S. tuberosum* Group Tuberosum cultivar.

The invention also comprises a tuber with deep yellow or orange flesh produced by a plant according to the invention. Further, the invention comprises a food product comprising such a tuber and the use of such a tuber for food purposes.

### LEGENDS TO THE FIGURES

Figure 1: Schematic representation of the 2255-bp genomic sequence of the potato beta-carotene hydroxylase 2 (CHY2) gene in monoploid 7322 (allele 1). The PCR product analysed for the presence of SNPs is indicated.
Figure 2: CAPS marker for analysis of dosage of CHY2 allele 3. 308-bp PCR product CHY2ex4F + Beta-R822, digested with AluI. Plants with orange fleshed tubers of diploids 'Papa Pura' and 'Andean Sunrise' both show a 1:1 ratio allele 3/other allele. White fleshed tetraploids 'Karnico' and 'Astarte' do not contain allele 3. Light yellow-fleshed tetraploid 'Marlen' shows a 3:1 ratio allele 3/other alleles.
Figure 3: Variation in flesh colour value in classes of tetraploid potato genotypes with 0x, 1x, 2x, 3, and 4x CHY2 allele 3. Flesh colour value equal to or below 5.5 is considered to correspond to white flesh, values above 5.5 are indicative of yellow flesh. Genetic background or observational inconsistencies may explain the few deviating cases where CHY2 allele 3 is absent, but the phenotypic value exceeds the threshold of 5.5
Figure 4: Schematic representation of the 7000-bp genomic sequence of the potato lycopene epsilon cyclase (LCYe) gene from BAC RH091F11. The PCR product analysed for the presence of SNPs is indicated.
Figure 5: QTL analysis of flesh colour value in the CxE population, showing a very small QTL on chromosome 12, at the position of the LCYe gene.
Figure 6: Relation between CHY2 allele 3 dosage, LCYe allele 2 dosage, and phenotypic value of the diploid CxE mapping population for tuber flesh colour.
Figure 7: Schematic representation of the genomic sequence (promoter and coding region) of the potato zeaxanthin epoxidase gene. (A) 11,000-bp sequence of allele 1. (B) 6,000-bp sequence of allele 2.
Figure 8: Analysis of IvP92-030 progeny for composition of CHY2 alleles (A) and ZEP alleles (B). IvP92-030-9 and IvP92-030-11 show orange tuber flesh colour. Both contain the dominant CHY2 allele 3 and are homozygous recessive for ZEP allele 1.
Figure 9: Relation between CHY2 allele 3 dosage, ZEP allele 1 dosage, and flesh colour value in the diploid CxE population.
Figure 10: Relation between CHY2 allele composition, ZEP allele composition, and (A) Yellow Area (measure for total yellow carotenoids), or (B) Zeaxanthin content (in µg/g fresh weight) in the diploid CxE population. Bars show mean values. Error bars show mean +/- 1.0 SE.
Figure 11: Results of quantitative RT-PCR. Relative expression level of the ZEP gene for CxE progeny homozygous for allele 1, heterozygous (allele 1 + allele 2), or homozygous for allele 2. ZEP allele 1 results in a lower expression level than ZEP allele 2.
Figure 12: Alignment of the genomic sequences of ZEP alleles 1 and 2.
Figure 13: Alignment of the deduced amino acid sequences of ZEP alleles 1 and 2.
Figure 14: Alignment of the deduced amino acid sequences of ZEP alleles 1 and 2, combined with the ZEP protein sequences of other Solanaceous species.

### DETAILED DESCRIPTION OF THE INVENTION

### Definitions

As used herein, the term "plant or part thereof" means any complete or partial plant, single cells and cell tissues such as plant cells that are intact in plants, cell clumps and tissue cultures from which potato plants can be regenerated. Examples of plant parts include, but are not limited to, single cells and tissues from pollen, ovules, leaves, embryos, roots, root tips, anthers, flowers, fruits, stems shoots, tubers, including potato tubers for consumption or 'seed tubers' for cultivation or clonal propagation, and seeds; as well as pollen, ovules, leaves, embryos, roots, root tips, anthers, flowers, fruits, stems, shoots, scions, rootstocks, seeds, protoplasts, calli, and the like.

As used herein, the term "population" means a genetically heterogeneous collection of plants sharing a common genetic derivation.

As used herein, the term "variety" is as defined in the UPOV treaty and refers to any plant grouping within a single botanical taxon of the lowest known rank, which grouping can be: (a) defined by the expression of the characteristics that results from a given genotype or combination of genotypes, (b) distinguished from any other plant grouping by the expression of at least one of the said characteristics, and (c) considered as a unit with regard to its suitability for being propagated unchanged.

The term "cultivar" (for cultivated variety) as used herein is defined as a variety that is not normally found in nature but that has been cultivated by humans, i.e. having a biological status other than a "wild" status, which "wild" status indicates the original non-cultivated, or natural state of a plant or accession. The term "cultivar" specifically relates to a potato plant having a ploidy level that is tetraploid. The term "cultivar" further includes, but is not limited to, semi-natural, semi-wild, weedy, traditional cultivar, landrace, breeding material, research material, breeder's line, synthetic population, hybrid, founder stock/base population, inbred line (parent of hybrid cultivar), segregating population, mutant/genetic stock, and advanced/improved cultivar.

As used herein, "crossing" means the fertilization of female plants (or gametes) by male plants (or gametes). The term "gamete" refers to the haploid or diploid reproductive cell (egg or sperm) produced in plants by meiosis, or by first or second restitution, or double reduction from a gametophyte and involved in sexual reproduction, during which two gametes of opposite sex fuse to form a diploid or polyploid zygote. The term generally includes reference to a pollen (including the sperm cell) and an ovule (including the ovum). "Crossing" therefore generally refers to the fertilization of ovules of one individual with pollen from another individual, whereas "selfing" refers to the fertilization of ovules of an individual with pollen from genetically the same individual.

The term "backcrossing" as used herein means the process wherein the plant resulting from a cross between two parental lines is crossed with one of its parental lines, wherein the parental line used in the backcross is referred to as the recurrent parent. Repeated backcrossing results in the genome becoming more and more similar to the recurrent parent, as far as this can be achieved given the level of homo- or heterozygosity of said parent.

As used herein, "selfing" is defined as refers to the process of self-fertilization wherein an individual is pollinated or fertilized with its own pollen.

The term "marker" as used herein means any indicator that is used in methods for inferring differences in characteristics of genomic sequences. Examples of such indicators are restriction fragment length polymorphism (RFLP) markers, amplified fragment length polymorphism (AFLP) markers, single nucleotide polymorphisms (SNPs), insertion mutations, microsatellite markers (SSRs), sequence-characterized amplified regions (SCARs), cleaved amplified polymorphic sequence (CAPS) markers or isozyme markers or combinations of the markers described herein which defines a specific genetic and chromosomal location.

As used herein, "gene" means a hereditary unit (often indicated by a sequence of DNA) that occupies a specific location on a chromosome and that contains the genetic instruction for the presence or absence of a particular phenotypic characteristics or trait in a plant.

As used herein, "locus" is defined as the genetic or physical position that a given gene occupies on a chromosome of a plant.

The term "allele(s)" as used herein means any of one or more alternative forms of a gene, all of which alleles relate to the presence or absence of a particular phenotypic trait or characteristic in a plant. In a diploid cell or organism, the two alleles of a given gene occupy corresponding loci on a pair of homologous chromosomes. It is in some instance more accurate to refer to "haplotypes" (i.e. an allele of a chromosomal segment) in stead of "allele", however, in these instances, the term "allele" should be understood to comprise the term "haplotype".

The term "heterozygous" as used herein, and confined to diploids, means a genetic condition existing when different alleles reside at corresponding loci on homologous chromosomes.

As used herein, and confined to diploids, "homozygous" is defined as a genetic condition existing when identical alleles reside at corresponding loci on homologous chromosomes.

As used herein, and confined to tetraploids, the term "nulliplex", "simplex", "duplex", "triplex" and "quadruplex", is defined as a genetic condition existing when a specific allele at a corresponding locus on corresponding homologous chromosomes is present 0, 1, 2, 3 or 4 times, respectively. At the tetraploid level the phenotypic effect associated with a recessive allele is only observed when the allele is present in quadruplex condition, whereas the phenotypic effect associated with a dominant allele is already observed when the allele is present in a simplex or higher condition.

The term "recessive allele" as used herein is defined as an allele whose phenotypic effect is not expressed in an individual organism which genotype is heterozygous for the allele, but is only expressed in an individual organism which genotype is homozygous or quadruplex for the recessive allele.

As used herein, the term "dominant allele" is defined as an allele whose phenotypic effect is expressed in an individual organism due to the presence of at least one copy, irrespective of the zygosity of the plant..

The terms "haploid", "diploid" and "tetraploid" as used herein are defined as having respectively one, two and four pairs of each chromosome in each cell (excluding reproductive cells).

The term "polymorphism" is defined as an allelic variant that occurs in a population. The polymorphism can be a single nucleotide difference present at a locus, or can be an insertion or deletion of one or a few nucleotides such as an indel, or the insertion of a large stretch of nucleotides such as a transposons insertion. As such, a single nucleotide polymorphism ("SNP") is characterized by the presence in a population of at least two, of the four nucleotides (i. e., adenosine, cytosine, guanosine or thymidine) at a particular homologous position or locus in a genome such as the potato genome. Accordingly, it will be recognized that, while the methods of the invention are exemplified primarily by the detection of SNPs, the disclosed methods or others known in the art similarly can be used to identify other polymorphisms in the exemplified genetic region or haplotype that is represented by the DNA sequence of the ZEP allele 1, as shown in Figure 12.

As used herein, the term "indel" is defined as a mutation in the DNA whereby one or more nucleotides are either inserted (Insertion) or deleted (DELetion), resulting in a net gain or loss of nucleotides. This includes any combination of insertions and deletions.

The term "haplotype" as used herein means a combination of alleles at multiple loci that are transmitted together on the same chromosome. This includes haplotypes referring to as few as two loci, and haplotypes referring to an entire chromosome depending on the number of recombination events that have occurred between a given set of loci. It also includes a set of polymorphisms on a single chromatid that are statistically associated with each other.

As used herein, the term "infer" or "inferring", when used in reference to assessing the presence of ZEP allele 1 or its recessiveness, means drawing a conclusion about the recessiveness of a ZEP allele in a plant or part thereof using a process of analyzing individually or in combination nucleotide occurrence(s) of one or more polymorphism(s) of the invention in a nucleic acid sample of the plant or part thereof, and comparing the individual or combination of nucleotide occurrence(s) of the polymorphism(s) to known relationships of nucleotide occurrence(s) of the ZEP allele recessiveness. As disclosed herein, the nucleotide occurrence(s) can be identified directly by examining the qualitative differences or quantitative differences in expression levels of nucleic acid molecules, or indirectly by examining (the expression level of) a polypeptide encoded by a particular gene, for example, the ZEP gene, when the polymorphism is associated with an amino acid change in the encoded polypeptide or has regulatory effects.

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in PCR amplification.

As used herein, the term "probe" means a single-stranded oligonucleotide sequence that will recognize and form a hydrogen-bonded duplex with a complementary sequence in a target nucleic acid sequence analyte or its cDNA derivative.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize non-specific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer.

Typically, stringent conditions will be those in which the salt concentration is less than about 1.0 M Na+ ion, typically about 0.01 to 1.0 M Na+ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel, F.M., Brent, R., Kingston, R.E., Moore, D.D.,Seidman, J.G., Smith, J.A., Struhl, K. eds. (1998) Current protocols in molecular biology. V.B. Chanda, series ed. New York: John Wiley & Sons.

### Description

The inventors found a hitherto uncharacterized ZEP allele that is recessive, that is only rarely found in tetraploid potato cultivars (allele frequency is below 1%) and that is characterized by several unique features in its nucleic acid sequence. The inventors surprisingly found that the level of zeaxanthin in a plant line can be increased by crossing and backcrossing plants that have at least one such a recessive ZEP allele. Furthermore, the inventors surprisingly found that increasing the level of zeaxanthin can be accomplished only in potato plants that are homozygous (diploid) or quadruplex (tetraploid) for the recessive ZEP allele. Furthermore, it was found that the presence of a dominant allele of the β-carotene hydroxylase (CHY2) gene caused an overall increase in the level of total carotenoids and that only when a potato plant contains the dominant allele of CHY2 in combination with homozygosity of the recessive ZEP allele, said plant produces tubers with deep-yellow or orange flesh caused by the accumulation of zeaxanthin. Only due to the finding of the epistatic interaction between the ZEP and the CHY2 gene and the requirement of homozygous recessive ZEP alleles it has been able to understand the lack of progress in breeding tetraploid potato cultivars with an increased level of zeaxanthin and/or orange tuber flesh.

Due to said hereditary requirements it now is understood why at the diploid level it is already difficult to breed for an increased level of zeaxanthin and/or orange tuber flesh. Moreover, only now it is understood why it is close to impossible to obtain these phenotypic traits in tetraploid potato cultivars which belong to *Solanum tuberosum* Group Tuberosum. First, the allele frequency of recessive ZEP alleles is so low (below 1%) in tetraploid potato germplasm that it is close to impossible that two parental clones will be crossed having at least one recessive ZEP allele, let alone that quadruplex plants will be formed. In other words: Although with the current knowledge from the invention a few tetraploid cultivars have been identified by the inventors, representing "hidden carriers" of one recessive ZEP allele, it is close to impossible that conventional breeding and selection would produce the quadruplex recessive offspring. To raise the number of alleles in plant material selection is required. Artificial selection among offspring genotypes can only be performed when it is known which offspring plants carry any number of recessive ZEP alleles. Since the presence of one, or two, or three recessive ZEP alleles will not result in any phenotypic effect, it was hitherto impossible to breed for the phenotype of plants with orange flesh tubers.

Only by using the current invention the presence of recessive ZEP alleles can be inferred in plant material. Only by using the current invention the suitable parental genotypes can be identified, having at least one recessive ZEP allele. Only by using the current invention the suitable offspring genotypes, having gained additional copies of the recessive ZEP allele, can be identified resulting in duplex, triplex or quadruplex genotypes, having two, three or four recessive ZEP alleles, respectively.

So far it has not been obvious for a person skilled in the art to understand the classical Mendelian inheritance of the phenotype of elevated level of zeaxanthin and/or orange flesh colour in potato tubers. The inheritance now surprisingly appears to be an example of a specific epistatic gene interaction termed "recessive epistasis". At the diploid level this results in the Mendelian 9:4:3 ratio. Example: the selfing of the dihybrid ZzCc results in the Mendelian 9:3:3:1 ratio for Z-C- : Z-cc : zzC- : zzcc genotypes. If Z represents a dominant ZEP allele, z being recessive, and if C represents a dominant CHY allele, c being recessive, then the following phenotypes and phenotypic ratios can be expected: 9 yellow tuber flesh : 3 white tuber flesh : 3 orange tuber flesh : 1 white tuber flesh.

At the tetraploid level the Mendelian ratios are different from those observed at the diploid level. For example, the crossing of two duplex parents will result in only 3% (Mendelian ratio = 35: 1) quadruplex offspring having homozygosity for the recessive ZEP allele, even ignoring the preferred requirement for the dominant CHY allele. Crossing of parental clones with fewer than two recessive ZEP alleles will almost never result in quadruplex offspring. It is therefore close to impossible to achieve orange potato plants by crossing alone. Thus, a method for increasing a plant line's zeaxanthin level is needed that preferably involves marker assisted selection of suitable parental genotypes, which would allow to confirm the Mendelian segregation ratios in the offspring, and thus to enhance the probability to obtain the desired phenotype, and wherein the selection of the offspring genotypes of these crossing comprises marker-assisted selection.

As mentioned before, an increased level of zeaxanthin and/or orange tuber flesh, can be found in diploid potato species. Specifically the native Andean material related to the diploid species *S. phureja* may be used as starting material to introgress said trait into *S. tuberosum* Group Tuberosum cultivars, requiring the invention of breeding strategies for said trait. Even if one would pursue this strategy of introgression breeding from *S. phureja* to tetraploid potato cultivars, the method disclosed in this invention has to be an integral element of said strategy. The current absence of tetraploid potato cultivars with an increased level of zeaxanthin and/or orange flesh proves that this alternative method using diploid material has also been prohibitively complicated.

Breeding of such lines is enhanced by selection with markers indicating these unique features.

Therefore, the inventors provide a method for increasing the level of zeaxanthin in a plant line, comprising: a) selecting at least two plants of a plant line with a genotype comprising at least one recessive ZEP allele, b) crossing said plants, c) selecting progeny from said crossing for a genotype comprising at least one recessive ZEP allele, d) selfing and /or backcrossing said progeny selected in step a) using said plant line as recurrent parent, e) selecting progeny resulting from the crossing in step d) for a genotype comprising at least one recessive ZEP allele, f) optionally repeating said steps of selfing and / or backcrossing and selection of steps d) and e) to provide a plant of a plant line having a genotype that is homozygous or quadruplex for said recessive ZEP allele, wherein at least one selection as performed in steps a), c) or e) is performed by marker-assisted selection and wherein said method optionally comprises increasing the ploidy or the use of unreduced gametes of any parental plants selected at step a) to obtain tetraploid offspring before or after conducting step b).

Any suitable method known in the art for crossing selected plants may be applied in the method according to the invention. This includes both *in vivo* and *in vitro* methods. A person skilled in the art will appreciate that *in vitro* techniques such as protoplast fusion or embryo rescue may be applied when deemed suitable.

Selected plants that are used for crossing purposes in the methods according to the invention may have any type of ploidy. For example, selected plants may be haploid, diploid or tetraploid. However, crossing diploid plants will only provide diploid offspring. Crossing a diploid plant with a tetraploid plant will result in triploid offspring that is sterile.

Thus, when plants are selected that are diploid, their ploidy must be increased to tetraploid level before they can be crossed with another tetraploid plant in the methods according to the invention. Methods for increasing the ploidy of a plant are well known in the art and can be readily applied by a person skilled in the art. For example, ploidy of a diploid plant for crossing purposes can be increased by using 2N gametes of said diploid plant . Ploidy can also be increased by inhibiting chromosome segregation during meiosis, for example by treating a diploid plant with colchicine. By applying such methods on a diploid plant, embryos or gametes are obtained that comprise double the usual number of chromosomes. Such embryos or gametes can then be used for crossing purposes.

Preferably, selected plants are crossed with each other using classical *in vivo* crossing methods that comprise one or more crossing steps including selfing. By applying such classical crossing steps characteristics of both the parents can be combined in the progeny. For example, a plant that provides a high yield can be crossed with a plant that contains large amounts of a certain nutrient. Such a crossing would provide progeny comprising both characteristics, i.e. plants that not only comprise large amounts of the nutrient but also provide high yields.

When applying backcrossing, F1 progeny is crossed with one of its high-yielding parents P to ensure that the characteristics of the F2 progeny resemble those of the high-yielding parent. For example, a selected diploid potato with orange flesh (i.e., containing high levels of zeaxanthin) is made tetraploid by using colchicine and then crossed with a selected high-yielding tetraploid potato cultivar, with the purpose of ultimately providing a high-yielding tetraploid progeny having orange flesh. Also selfing may be applied. Selected plants, either parent or progeny, are then crossed with themselves to produce inbred varieties for breeding. For example, selected specimens from the above mentioned F1 progeny are crossed with themselves to provide an F2 progeny from which specimens can be selected that have an increased level of zeaxanthin. However, potato is know to display severe inbreeding depression upon selfing, and selfing requires both male and female fertility which may not be the case, and therefore this strategy is cumbersome.

Preferred, therefore is a strategy of increasing the number of recessive alleles for ZEP in one parent plant by marker-assisted breeding, more preferably wherein said plant also comprises the dominant CHY2 allele, and thus would be capable of producing orange tubers, and then cross said parent plant with a high yielding variety in order to produce plants that have retained both characteristics from their parents.

When selecting and crossing a parental genotype in a method according to the invention, a marker is used to assist selection in at least one selection step. It is known in the art that markers, indicative for a certain trait or condition, can be found *in vivo* and *in vitro* at different biological levels. For example, markers can be found at peptide level or at gene level. At gene level, a marker can be detected at RNA level or DNA level. Preferably, in the present invention the presence of such a marker is detected at DNA level. Markers at DNA level are known in the art. For example, an SNP or haplotype that is unique for a certain gene or allele is indicative for a specific trait or condition associated with that gene or allele and is thus a suitable marker for that gene or allele.

It was found by the inventors that the ZEP allele 1 is a recessive allele and that plants having a genotype that is homozygous for this ZEP allele, and preferably in addition have a dominant CHY allele, have an increased level of zeaxanthin. The inventors further found that ZEP allele 1 comprises a unique indel in intron 4, including SNPs C422- and T426-. Other ZEP alleles can be discriminated from ZEP allele 1 by e.g. SNPs G83A, T99G, A113T, A154G, G165C, A180G, T209C, T231A, T375G, A384T, A389C, A415G, C422T and T426C or a combination of those. This amount of SNPs is sufficient to discriminate all 10 ZEP alleles that have been uncovered thus far. However, between the recessive ZEP 1 allele and any other of the ZEP alleles many more differences on nucleotide level are available. All these differences may be used to identify the ZEP allele 1 (with respect to any other ZEP allele). The nucleotide numbering of the SNPs is based on the nucleotide sequence of the PCR product of primers AWZEP9 and AWZEP10. In order to correlate this numbering with the numbering used in Fig. 12 in which the nucleotide sequence of ZEP allele 1 is presented, all figures should be added to 6802. Thus, the SNP identified as G83A is to be found on nucleotide 6885 in Figure 12. Importantly, it was further found that ZEP allele 1 comprises a unique transposon insertion in intron 1, and a unique 49-base pairs indel in intron 4. Thus, each of these polymorphisms can be used as a marker to assist in at least one selection step in the methods according to the invention. For example, a plant comprising a ZEP allele is selected based on the presence of the 49-base pairs deletion in intron 4 and is crossed with another plant comprising a ZEP allele that was selected based on the presence of the unique transposon insertion. The progeny of that crossing is then also selected on presence of the ZEP allele based on the presence of at least one of the markers. The selected progeny can be used in subsequent crosses with any parent plant having the genotype comprising the 49-base pairs deletion in intron 4, or selfed.

Levels of zeaxanthin in a plant line can be further increased by the method according to the invention, when the progeny that is provided comprises a genotype that is homozygous or quadruplex for the recessive ZEP allele and further comprises at least one dominant CHY2-allele, identified herein as CHY2-allele 3. This dominant CHY2-gene also comprises SNPs. It was found that SNP T142C is indicative for the presence of said CHY2-allele 3. Thus, the final plant line that is obtained by a method according to the invention preferably has a genotype that is quadruplex for the recessive ZEP allele 1 and comprises at least one CHY2 allele comprising SNP T142C.

It will be clear to the skilled person that any method suitable for detecting a nucleotide change may be applied when selecting a plant or plant line in the method according to the invention. Methods for detecting a nucleotide change can utilize one or more oligonucleotide probes or primers that selectively hybridize to a target polynucleotide which contains one or more SNP positions or other markers. Such probes or primers include, for example, an amplification primer pair. Probes useful in practicing a method of the invention can include, for example, an oligonucleotide that is complementary to and spans a portion of the target polynucleotide, including the position of the marker, wherein the presence or absence of a specific nucleotide at the position (e.g, an SNP, an indel or a transposon insertion) is detected by the presence or absence of selective hybridization of the probe. Such a method can further include contacting the target polynucleotide and hybridized oligonucleotide with an endonuclease, and detecting the presence or absence of a cleavage product of the probe, depending on whether the nucleotide occurrence at the marker site is complementary to the corresponding nucleotide of the probe. A pair of probes that specifically hybridize upstream and adjacent and downstream and adjacent to the site of the marker, wherein one of the probes includes a nucleotide complementary to a nucleotide occurrence of the marker, also can be used in an oligonucleotide ligation assay, wherein the presence or absence of a ligation product is indicative of a specific nucleotide occurrence at the marker site. An oligonucleotide also can be useful as a primer, for example, for a primer extension reaction, wherein the product (or absence of a product) of the extension reaction is indicative of the nucleotide occurrence. In addition, a primer pair useful for amplifying a portion of the target polynucleotide including the marker site can be useful, wherein the amplification product is examined to determine the nucleotide occurrence at the marker site.

Where the particular nucleotide occurrence of a marker, or nucleotide occurrences of a haplotype, is such that the nucleotide occurrence results in an amino acid change in an encoded polypeptide, the nucleotide occurrence can be identified indirectly by detecting the particular amino acid in the polypeptide. The method for determining the amino acid will depend, for example, on the structure of the polypeptide or on the position of the amino acid in the polypeptide. Where the polypeptide contains only a single occurrence of an amino acid encoded by the particular polymorphism, the polypeptide can be examined for the presence or absence of the amino acid. For example, where the amino acid is at or near the amino terminus or the carboxy terminus of the polypeptide, simple sequencing of the terminal amino acids can be performed. Alternatively, the polypeptide can be treated with one or more enzymes and a peptide fragment containing the amino acid position of interest can be examined, for example, by sequencing the peptide, or by detecting a particular migration of the peptide following electrophoresis. Where the particular amino acid comprises an epitope of the polypeptide, the specific binding, or absence thereof, of an antibody specific for the epitope can be detected. Other methods for detecting a particular amino acid in a polypeptide or peptide fragment thereof are well known and can be selected based, for example, on convenience or availability of equipment such as a mass-spectrometer, capillary electrophoresis system, magnetic resonance imaging equipment, and the like.

The marker-assisted selection steps in the methods of the invention can in principle be performed by applying any nucleic acid amplification method, such as the Polymerase Chain Reaction (PCR; Mullis 1987, U.S. Pat. No. 4,683,195, 4,683,202, en 4,800,159) or by using amplification reactions such as Ligase Chain Reaction (LCR; Barany 1991, Proc. Natl. Acad. Sci. USA 88:189-193; EP Appl. No., 320,308), Self-Sustained Sequence Replication (3SR; Guatelli et al., 1990, Proc. Natl. Acad. Sci. USA 87:1874-1878), Strand Displacement Amplification (SDA; U.S. Pat. Nos. 5,270,184, en 5,455,166), Transcriptional Amplification System (TAS; Kwoh et al., Proc. Natl. Acad. Sci. USA 86:1173-1177), Q-Beta Replicase (Lizardi et al., 1988, Bio/Technology 6:1197), Rolling Circle Amplification (RCA; U.S. Pat. No. 5,871,921), Nucleic Acid Sequence Based Amplification (NASBA), Cleavage Fragment Length Polymorphism (U.S. Pat. No. 5,719,028), Isothermal and Chimeric Primer-initiated Amplification of Nucleic Acid (ICAN), Ramification-extension Amplification Method (RAM; U.S. Pat. Nos. 5,719,028 and 5,942,391) or other suitable methods for amplification of DNA.

In order to amplify DNA with a small number of mismatches to one or more of the amplification primers, an amplification reaction may be performed under conditions of reduced stringency (e.g. a PCR amplification using an annealing temperature of 38°C, or the presence of 3.5 mM MgCl₂). The person skilled in the art will be able to select conditions of suitable stringency.

The detection of the amplification products can in principle be accomplished by any suitable method known in the art. The detection fragments may be directly stained or labeled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes.

Alternatively, the DNA fragments may be detected by incorporation of labeled dNTP bases into the synthesized DNA fragments. Detection labels which may be associated with nucleotide bases include e.g. fluorescein, cyanine dye or BrdU.

When using a probe-based detection system, a suitable detection procedure for use in the present invention may for example comprise an enzyme immunoassay (EIA) format.

Probes useful for the detection of the target DNA as disclosed herein preferably bind only to at least a part of the DNA sequence region as amplified by the DNA amplification procedure. Those of skill in the art can prepare suitable probes for detection based on the nucleotide sequence of the target DNA without undue experimentation. Also the complementary sequences of the target DNA may suitably be used as detection probes in a method of the invention, provided that such a complementary strand is amplified in the amplification reaction employed.

Any suitable method for screening the nucleic acids of a plant or part thereof for the presence or absence of polymorphisms is considered to be part of the methods according to the invention. Such screening methods include, but are not limited to: DNA sequencing, restriction fragment length polymorphism (RFLP) analysis, amplified fragment length polymorphism (AFLP) analysis; heteroduplex analysis, single strand conformational polymorphism (SSCP) analysis, denaturing gradient gel electrophoresis (DGGE), real time PCR analysis (e.g. Taqman®), temperature gradient gel electrophoresis (TGGE), primer extension, allele-specific hybridization, and INVADER® genetic analysis assays, cleavage fragment length polymorphism (CFLP) analysis, sequence-characterized amplified region (SCAR) analysis, cleaved amplified polymorphic sequence (CAPS) analysis

The development of primers and probes useful for the detection of polymorphic positions in a nucleic acid is within the realm of ordinary skill (see for instance Sambrook, J. et al., 2001).

By using standard DNA technology it is possible to produce probes and primers that directly or indirectly hybridize to the DNA samples to be tested or cDNA produced from RNA by reverse transcription, and which can be used in assays for the detection of the markers. Nucleic acid amplification techniques allow the amplification of fragments of nucleic acids, which may be present in very low amounts.

In order to develop nucleic acid-based detection methods, the SNP-specific sequences must be determined for which primers or probes may then be developed. To detect the SNPs by nucleic acid amplification and/or probe hybridization, the nucleic acid may be isolated from any raw sample material, optionally reverse transcribed into cDNA and directly cloned and/or sequenced. DNA and RNA isolation kits are commercially available from for instance QIAGEN GmbH, Hilden, Germany, or Roche Diagnostics, a division of F. Hoffmann-La Roche Ltd, Basel, Switzerland. Nucleic acid-based detection of insertions or deletions can be accomplished accordingly.

A sample useful for practicing a method of the invention can be any biological sample from a plant or a part thereof that contains nucleic acid molecules, including portions of the allele sequences to be examined, or corresponding encoded polypeptides, depending on the particular method. As such, the sample can be a cell or tissue sample. As some of the markers are located in a non-coding region, the nucleic acid sample generally is a deoxyribonucleic acid (DNA) sample, particularly genomic DNA or an amplification product thereof. However, where hetero-nuclear ribonucleic acid (RNA), which includes unspliced mRNA precursor RNA molecules, is available, a cDNA or amplification product thereof can be used. The nucleic acid sample can thus be DNA or RNA, or products derived therefrom such as, for example, amplification products.

Using either the cloned nucleic acid as a hybridization probe, using sequence information derived from the clone, or by designing degenerative primers based on the sequence of the SNP and its flanking sequences, nucleic acid hybridization probes and/or nucleic acid amplification primers may be designed an used in a detection assay for detecting the ZEP SNPs in a sample as defined herein.

The DNA, or alternatively, the cDNA may be PCR amplified by using for instance Pfu and Taq DNA polymerases and amplification primers specific for the SNP DNA sequences. Also complete commercially available systems may be used for PCR (e.g. available form various suppliers such as Roche Diagnostics). A suitable method may for instance include mixing into a suitable aqueous buffering system (e.g. a commercially available PCR buffer) a suitable amount of total DNA as a template (e.g. 1 to 5 µg), a suitable amount (e.g. 10 pmol) of a pair of bi-directional amplification primers, a suitable amount of dNTPs and the DNA polymerase, denaturing the nucleic acids by boiling for 1 min, and performing a cycling reaction of around 10-50 alternating cycles of stringent primer hybridization, strand elongation and denaturing, at suitable temperatures to obtain DNA copies of the DNA template as amplification product. The amount of copies produced upon a certain number of cycles correlates directly to the amount of target DNA in the DNA template.

The skilled person is well aware of the available quantitative PCR methods presently available from commercial suppliers to quantify the amount of target DNA in the template. The term "hybridization signal" as used herein inter alia refers to the amount of amplification product produced upon a certain number of cycles and thus to the amount of target DNA available as template in the reaction.

In order to amplify a nucleic acid with a small number of mismatches to one or more of the amplification primers, an amplification reaction may be performed under conditions of reduced stringency (e.g. a PCR amplification using an annealing temperature of 38°C, or the presence of 3.5 mM MgCl₂). The person skilled in the art will be able to select conditions of suitable stringency.

The primers herein are selected to be "substantially" complementary (i.e. at least 65%, more preferably at least 80% perfectly complementary) to their target regions present on the different strands of each specific sequence to be amplified. It is possible to use primer sequences containing e.g. inositol residues or ambiguous bases or even primers that contain one or more mismatches when compared to the target sequence. In general, sequences that exhibit at least 65%, more preferably at least 80% homology with the target DNA or RNA oligonucleotide sequences are considered suitable for use in a method of the present invention. Sequence mismatches are also not critical when using low stringency hybridization conditions.

The detection of the amplification products can in principle be accomplished by any suitable method known in the art. The amplified fragments may be directly stained or labeled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes.

Alternatively, the DNA or RNA fragments may be detected by incorporation of labeled dNTP bases into the synthesized fragments. Detection labels which may be associated with nucleotide bases include e.g. fluorescein, cyanine dye, digoxigenin (DIG) or bromodeoxyuridine (BrdU).

Other methods of analysing the nucleic acid suitably comprise the use of a primer extension assay; a Taqman® PCR; a differential hybridization assay; an assay which detects allele-specific enzyme cleavage; and/or allele-specific PCR.

When using a probe-based detection system, a suitable detection procedure for use in the present invention may for example comprise an enzyme immunoassay (EIA) format (Jacobs et al., 1997, J Clin Microbiol 35:791-795). For performing a detection by manner of the EIA procedure, either the forward or the reverse primer used in the amplification reaction may comprise a capturing group, such as a biotin group for immobilization of target DNA PCR amplicons on e.g. a streptavidin coated microtiter plate wells or streptavidin coated Dynabeads® (Dynal Biotech, Oslo, Norway) for subsequent EIA detection of target DNA amplicons. The skilled person will understand that other groups for immobilization of target DNA PCR amplicons in an EIA format may be employed.

Probes useful for the detection of the target nucleic acid sequences preferably bind only to at least a part of the nucleic acid sequence region as amplified by the nucleic acid amplification procedure. Those of skill in the art can prepare suitable probes for detection based on the nucleotide sequence of the target nucleic acid without undue experimentation as set out herein. Also the complementary nucleotide sequences, whether DNA or RNA or chemically synthesized analogues, of the target nucleic acid may suitably be used as type-specific detection probes in a method of the invention, provided that such a complementary strand is amplified in the amplification reaction employed.

Suitable detection procedures for use herein may for example comprise immobilization of the amplicons and probing the nucleic acid sequences thereof by e.g. Northern and Southern blotting. Other formats may comprise an EIA format as described above. To facilitate the detection of binding, the specific amplicon detection probes may comprise a label moiety such as a fluorophore, a chromophore, an enzyme or a radio-label, so as to facilitate monitoring of binding of the probes to the reaction product of the amplification reaction. Such labels are well known to those skilled in the art and include, for example, fluorescein isothiocyanate (FITC), 6-galactosidase, horseradish peroxidase, streptavidin, biotin, digoxigenin, ³⁵S, ¹⁴C, ³²P or ¹²⁵I. Other examples will be apparent to those skilled in the art.

Detection may also be performed by a so-called reverse line blot (RLB) assay, such as for instance described by Van den Brule *et al.* (2002). For this purpose RLB probes are preferably synthesized with a 5' amino group for subsequent immobilization on e.g. carboxyl coated nylon membranes. The advantage of an RLB format is the ease of the system and its speed, thus allowing for high throughput sample processing.

The use of nucleic acid probes for the detection of RNA or DNA fragments is well known in the art. Mostly these procedures comprise the hybridization of the target nucleic acid with the probe followed by post-hybridization washings. Specificity is typically the function of post-hybridization washes, the critical factors being the ionic strength and temperature of the final wash solution. For nucleic acid hybrids, the Tm can be approximated from the equation of Meinkoth and Wahl (1984): Tm = 81.5 °C + 16.6 (log M) + 0.41 (% GC)-0.61 (% form)-500/L; where M is the molarity of monovalent cations, % GC is the percentage of guanosine and cytosine nucleotides in the nucleic acid, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1 °C for each 1 % of mismatching; thus, the hybridization and/or wash conditions can be adjusted to hybridize to sequences of the desired identity. For example, if sequences with > 90% identity are sought, the Tm can be decreased 10°C. Generally, stringent conditions are selected to be about 5 °C lower than the thermal melting point (Tm) for the specific sequence and its complement at a defined ionic strength and pH. However, severely stringent conditions can utilize a hybridization and/or wash at 1, 2, 3, or 4 °C lower than the thermal melting point (Tm); moderately stringent conditions can utilize a hybridization and/or wash at 6, 7, 8, 9, or 10 °C lower than the thermal melting point (Tm); low stringency conditions can utilize a hybridization and/or wash at 11, 12, 13, 14, 15, or 20 °C lower than the thermal melting point (Tm). Using the equation, hybridization and wash compositions, and desired Tm, those of ordinary skill will understand that variations in the stringency of hybridization and/or wash solutions are inherently described. If the desired degree of mismatching results in a Tm of less than 45 °C (aqueous solution) or 32 °C (formamide solution) it is preferred to increase the SSC concentration so that a higher temperature can be used. An extensive guide to the hybridization of nucleic acids is found in Tijssen, 1993 supra; Ausubel *et al*., 1998 supra.

Such detection methods can readily be applied for the purpose of selecting a plant of part thereof with one ore more ZEP allele 1 alleles and/or with the CHY2 dominant allele.

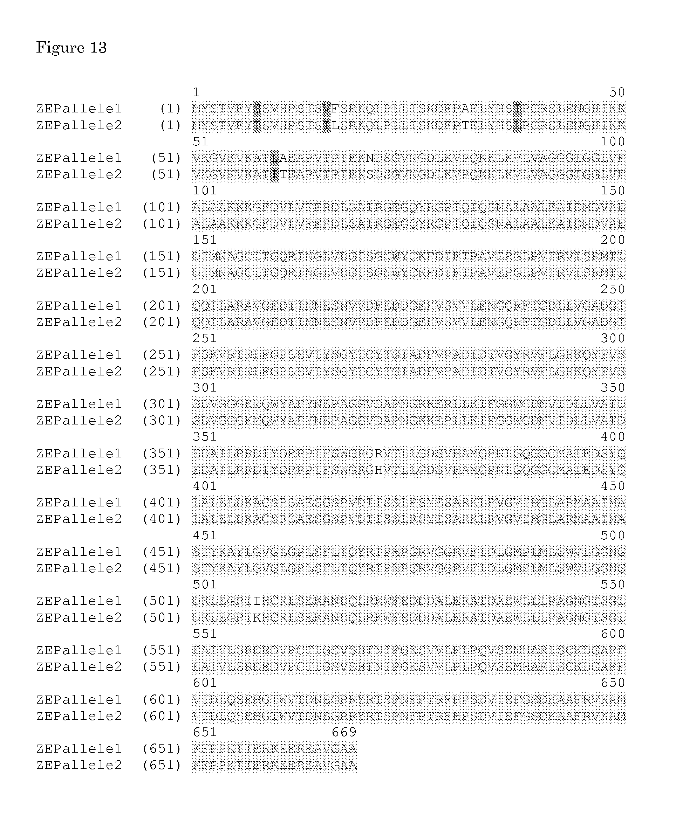

The inventors found a hitherto uncharacterized ZEP allele that is recessive, that is only rarely found in potato cultivars (allele frequency is below 1%) and that is characterized by several unique features in its nucleic acid sequence. In potato the gene is composed of 669 amino acids. In Figure 13 the protein alignment is shown of ZEP allele 1 and ZEP allele 2. From this alignment a large number of amino acid polymorphisms can be inferred, such as T8S, I16V, L17F, T32A, L38I, I60L, T61A, S72N, H372R and K508I, which are just only the amino acid substitutions from the dominant ZEP allele 2 to the recessive ZEP allele 1. There is no evidence that any of these amino acid changes have an influence on the enzymatic function of ZEP, but each polymorphism can be exploited to infer the allelic composition of potato genotypes. As shown in Table 5, at least nine different alleles have been identified on the basis of the re-sequencing of allelic variants, which have been obtained by PCR amplification of a genomic region demarcated by PCR primers AWZEP9 + AWZEP10 listed in Table 1. Hence the number of amino acid polymorphisms, as well as the number of DNA polymorphisms is much larger in the potato gene pool than only those polymorphisms between ZEP allele 1 and ZEP allele 2.

Hence in any method that is making use of crossing parents with at least one ZEP allele 1 and without any ZEP allele 2 will identification of different amino acid polymorphisms or different DNA polymorphisms needs to be performed to enable the inferring of the presence of ZEP allele 1. Except the SNPs listed in Table 5, there will be many more differences between the individual ZEP alleles in a one-to-one comparison. However, currently no attempt has been made to list all of these, since they will be easily detectable by a person skilled in the art by cloning and sequencing the individual alleles, which can be done on basis of the differences denoted in Table 5. Furthermore, any other DNA sequence polymorphism upstream or downstream and belonging to the same haplotype of any ZEP allele could be exploited to infer the allelic composition of potato genotypes, and to infer the presence of the recessive ZEP allele 1.

Yet a more reliable and robust polymorphism, in comparison to the SNP differences between the individual ZEP alleles is the unique presence of a 4102-bp transposon-like sequence which is diagnostic for ZEP allele 1. This transposon-like insertion has been integrated in the sequence of intron 1 of the ZEP gene, and the insert also has caused a target site duplication of 18 bp. The presence of such a long insertion in the intron is likely to affect splicing efficiency and therefore reducing the transcript level, and therefore the protein level. The conclusion that this transposon-like insertion within intron 1, is the causative event changing a dominant ZEP allele into a recessive ZEP allele should not be taken as a conclusion excluding any other molecular events in any other allele, affecting the functionality of any allele. Sofar, we are not aware of any other ZEP allele apart from ZEP allele 1 that acts in a recessive manner.

This transposon-like insertion is therefore the most obvious polymorphism allowing differentiation between the recessive allele 1 and any other ZEP allele. Although assaying for the presence of this insertion is the preferred method to discriminate between ZEP allele 1 and any other ZEP allele, it is certainly not the only way and any number of other polymorphisms upstream or downstream the ZEP gene, allowing to infer the presence of the specific haplotype known to encode the recessive ZEP allele 1, may be used by the person skilled in the art.

It will be appreciated by a person skilled in the art that any of the abovementioned detection methods is suitable for testing a plant or part thereof.

In another aspect, the invention provides the use of a marker for selecting a plant or part thereof, including a seed and/or a tuber, with a genotype comprising at least one recessive ZEP allele, wherein said marker is a polymorphism selected from the group consisting of G83A, T99G, A113T, A154G, G165C, A180G, T209C, T231A, T375G, A384T, A389C, A415G, C422-, C422T, T426- and T426C, the transposon insertion in intron 1 of the nucleic acid sequence of ZEP allele 1 in Figure 13 or the 49-base pairs indel in exon 4 of the nucleic acid sequence of ZEP allele 1 in Figure 13. For example, a plant or seed thereof or tuber is tested for the presence of the transposon insertion by application of a probe and consequent DNA amplification by PCR. Presence of the insertion indicates that the DNA comprises one or more of the recessive ZEP alleles 1 . The plant, tuber or seed may then be selected for further purposes.

For assessing whether a plant possesses a dominant CHY2 allele, a similar use of markers is contemplated. From the experimental part, especially Table 2, it can be derived that there are (at least) 12 different alleles for the CHY2 gene, of which allele no. 3 is the dominant allele. This allele is uniquely characterized by the presence of the SNP T142C, but any difference with any of the other CHY2 alleles (such as the SNPs listed in Table 2) can be used to identify the dominant allele with respect to any of the other CHY2 alleles. Further, a specific primer sequence (yellowF1: 5'TAATTACTCCATTCTTTTGCT) for the dominant allele has been disclosed by Brown et al. (2006). This primer can also be used for the identification of the allele.

By using the methods, markers or probes according to the invention a tetraploid potato having an increased zeaxanthin content is obtained, wherein the genotype of that potato comprises quadruplex recessive ZEP alleles comprising the nucleic acid sequence of ZEP allele 1 in Figure 12. In a further embodiment, a tetraploid potato is obtained, wherein the genotype of that potato comprises quadruplex recessive ZEP alleles comprising the nucleic acid sequence of ZEP allele 1 in Figure 12 and at least one CHY2 allele comprising SNP T142C. Preferably, said potato is a *S. tuberosum* Group Tuberosum cultivar.

Such a potato of which the tubers have orange flesh is not only suitable for preventing macula degeneration, but it is especially suitable for decorative foods. Potato tubers are commonly used as foodstuff in different forms: mashed potatoes, whole baked potatoes, boiled and steamed potatoes, grated potatoes, fried whole potatoes, potato chips, potato crisps, potato flakes, French fries, roasted potato cubes, rösti, and potato pancakes. Further, potatoes are used in other dishes (such as soups) to lend these dishes a waxy mouthfeel. Finally, potatoes can be used to brew alcoholic liquids (such as vodka). It is submitted that potato tubers with a deep-yellow or orange flesh are useful in the above mentioned foodstuffs for decorative purposes, i.e. in cases where a colourful dish needs to be prepared. Such a use would be especially applicable in occasions where orange has a specific meaning, like in the Ukraine, in the Netherlands , Northern Ireland and Ivory Coast. Further, the orange colour is favored by many political movements and it represents several social and historical groups and sport teams (see Wikipedia).

The invention is exemplified by the following example. This example is intended to illustrate the invention, without limiting the scope thereof in any way.

### EXAMPLE

This example describes DNA polymorphisms in three candidate genes involved in the carotenoid pathway in monoploid, diploid and tetraploid potato genotypes, and explains the inheritance of the level of various carotenoids and the corresponding tuber flesh colour phenotypes, i.e. white, yellow and orange potato tuber flesh colour.

### Materials and methods

### Plant materials

For sequence analyses DNA was used from five monoploid potato genotypes: 7322 (H7322 or AM79.7322, originally from G. Wenzel, Institüt für Genetik, Grünbach, Germany, see: de Vries *et al.* 1987; Hovenkamp-Hermelink *et al.* 1988), M5 and M38 (851-5 and 851-38, Uijtewaal 1987), M47 and M133 (1022M-47 and 1022M-133, Hoogkamp *et al.* 2000). DNA was obtained from 20 monoploid *S. phureja* and *S. chacoense* clones from Richard Veilleux (Blacksburg, Virginia, USA, see Lightbourn and Veilleux, 2007). DNA was isolated from eleven diploid genotypes: C (USW5337.3, Hanneman and Peloquin 1967), E (77.2102.37, Jacobsen 1980), RH88-025-50 and RH90-038-21 (Park *et al.* 2005), RH89-039-16 and SH83-92-488 (Rouppe van der Voort, 1997; van Os *et al.,* 2006), 87.1024/2 and 87.1029/31 (Jacobsen *et al.* 1989), G254 (Uijtewaal *et al.,* 1987), R5 (EJ92-6486-19, from cross 87.1024/2 x EJ91-6104-19) and 413 (transformant of interdihaploid H²260; De Vries-Uijtewaal *et al.,* 1989; Binding *et al.,* 1978).

Three diploid orange-fleshed genotypes were analysed: cultivars Papa Pura and Andean Sunrise (provided by Agrico Research BV), and *S. phureja* Yema de Huevo (obtained from Enrique Ritter, Vitoria, Spain, see Ritter *et al.* 2008). Two diploid populations were analysed in which orange-fleshed progeny segregated: the CxE population (Jacobs *et al.* 1995) and the IvP92-030 population (cross G254 x SUH2293, from Ronald Hutten). Furthermore, orange-fleshed diploid genotype IvP01-84-19 from the cross 96-4622-20 x IvP92-027-9 (Ronald Hutten) was included. Additionally, a set of 225 tetraploid cultivars was used (D'hoop *et al.* 2008; D'hoop 2009). In this set no genotypes with orange-fleshed tubers were present. Flesh colour of the tetraploids was determined in a field experiment in 2006 (D'hoop 2009). Flesh colour values were on an ordinal scale ranging from 4 (= white) to 9 (= orange).

### DNA isolation

Genomic DNA from the monoploid and diploid genotypes was isolated from leaf tissue according to the CTAB method from Rogers and Bendich (1988). DNA from the tetraploid cultivars was isolated according to Van der Beek *et al.* (1992).

### PCR amplification and sequencing

Amplicons for sequencing were generated from 50 ng genomic DNA template. PCR amplifications were performed in 50 µl or 25 µl reactions using 1 u of Taq polymerase, 1x reaction buffer, 200 nM dNTP and 250 nM of each primer. Standard cycling conditions were: 4 minutes initial denaturation at 94°C, followed by 35 cycles of 30 seconds denaturation at 94°C, 30 seconds annealing at 55°C and 30 seconds to 1 minute extension at 72°C. Reactions were finished by 7 minutes incubation at 72°C. Most PCRs were performed with SuperTaq™ Polymerase buffer and enzyme (Ambion Inc., TX, USA). PCR products were examined for quality on ethidium bromide-stained agarose gels. PCR products were directly sequenced on ABI377 or ABI3700 sequencers at Greenomics (Wageningen University and Research Centre) using the dideoxy chain-termination method and ABI PRISM® Reaction Kit (Applied Biosystems, CA, USA). One or both of the amplification primers were used as sequencing primers. The used primers and their respective nucleic acid sequences are listed in Table 1.

**Table 1. List of primers and their respective nucleic acid sequences**

| Primer | Sequence (5'→3') |
|---|---|
| CHY2ex4F | CCATAGACCAAGAGAAGGACC |
| Beta-R822 | GAAAGTAAGGCACGTTGGCAAT |
| AWLCYe1 | AAAAGATGCAATGCCATTCGAT |
| AWLCYe2 | GAAATACTCGGGGTACTTGAAC |
| AWZEP9 | GTGGTTCTTGAGAATGGACAAC |
| AWZEP10 | CACCAGCTGGTTCATTGTAAAA |
| AWZEP20 | TCATTCATAATTGTATCCTCCC |
| AWZEP25 | CTGGCTGCATCACTGGTCAAAG |
| AWZEPGW1 | TTCTGTGGAACCTTCAAATCACCGTTA |
| AWZEPGW2 | GCCCATTTTCCAAGCTCCTACAAGGTA |
| StZEP_RT_F | AAGTGCCGAGTCAGGAAGCC |
| StZEP_RT_R | CAAGTCCGACGCCAAGATAAGC |

### SNP analysis

Trace files were analysed with the Invitrogen™ Vector NTI® software package (Life Technologies Corporation, CA, USA).

### Genetic mapping and QTL analysis in the CxE population

Based on an earlier version of the C x E genetic map (Celis-Gamboa 2002) a simplified map was made using mapping software Joinmap 4.0 ^{®} (Van Ooijen 2006) based on 94 individuals with few additional markers (A. Kumari, unpublished results). QTL analysis of quantitative data was performed using the software package MapQTL® Version 5.0 (Van Ooijen 2004).

### Cloning of ZEP promoter sequence

The promoter sequence of ZEP allele 1 was obtained by Genome Walking using the Universal GenomeWalker™ kit (Clontech Laboratories Inc., CA, USA) and the BD Advantage™ 2 PCR Enzyme System (BD Biosciences, CA, USA). DNA from diploid genotype R5 (homozygous for ZEP allele 1) was used as template. Four libraries were made using *Dra*I*, Eco*RV*, Stu*I and *Sca*I enzymes. The first PCR was performed with primer AP1 (see GenomeWalker™ kit) and gene-specific primer AWZEPGW1 (5'-TTCTGTGGAACCTTCAAATCACCGTTA-3'). The nested PCR was performed with primer AP2 (see GenomeWalker™ kit) and gene-specific primer AWZEPGW2 (5'-GCCCATTTTCCAAGCTCCTACAAGGTA-3'). The *Dra*I*-* and *Stu*I-libraries yielded a 1.1-kb and 1.8-kb PCR fragment, respectively. The 1.8-kb PCR fragment of the *Stu*I library was sequenced, and proved to contain a *Dra*I restriction site at the expected position.

### Cloning intron 1 of ZEP allele 1

To obtain the intron 1 sequence of ZEP allele 1 primers AWZEP25 (5'-CTGGCTGCATCACTGGTCAAAG-3') and AWZEP20 (5'-TCATTCATAATTGTATCCTCCC-3') were used. The Expand High Fidelity PCR System (Roche Diagnostics Corporation, IN, USA) was used to obtain the 4.7-kb PCR fragment. This fragment was cloned into pGEM®-Teasy (Promega Corporation, WI, USA). Plasmid DNA was isolated using the Promega Wizard® Plus minipreps DNA Purification system (Promega Corporation, WI, USA). DNA from three independent colonies was first sequenced using the T7, AWZEP25 and AWZEP20 primers, and subsequently with primers designed on the obtained sequences.

### Measurement of carotenoids

Carotenoids were extracted and analyzed by HPLC with photodiode array (PDA) detection, according to the protocol described by Bino et al. (2005). In short, 0.5 g FW of ground and frozen tuber material was extracted with methanol/chloroform/ 1 M NaCl in 50 mM Tris (pH 7.4) in a ratio of 2.5: 2: 2.5 (v:v:v) containing 0.1% butylated hydroxytoluene (BHT). After centrifugation, the samples were re-extracted with 1 ml chloroform (+ BHT). The chloroform fractions were combined, dried under a flow of N₂ gas and taken up in ethyl acetate containing 0.1% BHT. Carotenoids present in the extracts were separated by HPLC using a YMC-Pack reverse-phase C30 column and analyzed by PDA detection with wavelength range set from 240 to 700 nm. Eluting compounds were identified based on their absorbance spectra and coelution with commercially available authentic standards (neoxanthin, violaxanthin, antheraxanthin, lutein, zeaxanthin, β-cryptoxanthin, ε-carotene, α-carotene, 6-carotene, ξ-carotene, δ-carotene, prolycopene and all-trans lycopene. Limit of detection was about 5 µg/100 g FW and technical variation (6 independent extractions and analyses of the same tuber powder) was less than 8%.

### Quantitative RT-PCR

Total RNA of 23 selected genotypes of the CxE population were isolated from dormant tubers as described by Bachem *et al.* (1998). mRNA was purified using the RNeasy mini kit (Qiagen GmbH, Germany) and reverse transcribed using the iScript™ cDNA synthesis kit from Bio-Rad (Bio-Rad Laboratories Inc., CA, USA). Relative expression level of the ZEP locus was determined by real-time quantitative reverse transcriptase PCR (qRT-PCR) on an iQ™ detection system (Bio-Rad) according to the Bio-Rad iQ™ SYBR® Green Supermix protocol. The primer sequences used for the analysis were StZEP_RT_F (5'-AAGTGCCGAGTCAGGAAGCC-3') from exon 7 and StZEP_RT_R (5'-CAAGTCCGACGCCAAGATAAGC-3') from exon 8. Potato elongation factor 1-α primers (EF1α) were used for relative quantification (Nicot *et al.* 2005). Relative quantification of the target RNA expression level was performed using Bio-Rad's iQ™ 5 analysis program.

### Results

### SNP analysis of the beta-carotene hydroxylase gene (CHY2)

Expression studies using the 44K POCI array (Kloosterman *et al.* 2008) resulted in the identification of an eQTL for yellow flesh colour. Further analysis indicated that in the diploid CxE population the parent C-specific allele of the CHY2 gene was correlated with yellow flesh. This allele shows higher expression than the other alleles segregating in the CxE population, indicating this is a dominant allele, as was previously observed by Brown *et al.* (2006). Both these studies were performed in diploid genotypes. Sequence analyses on DNA of a set of 225 tetraploid potato cultivars (D'hoop *et al.* 2009) were performed to analyze the effect of CHY2 alleles in a tetraploid background. First however, the complete genomic sequence of the CHY2 allele in monohaploid potato genotype 7322 was determined (Figure 1), as only mRNA and EST sequences were known for the potato CHY2 gene. Next, direct sequence analyses of a PCR fragment obtained with primers CHY2ex4F and Beta-R822, spanning exon 4, intron 4 and exon 5 of the CHY2 gene were performed. DNA of four other potato monoploids, 20 *S. phureja* and *S. chacoense* monoploids, and 11 diploids was used. The results of the sequence analyses of the PCR fragment are listed in Table 2. From these sequences in total 8 different haplotypes could be determined (Table 2; alleles 1-6B and 12).

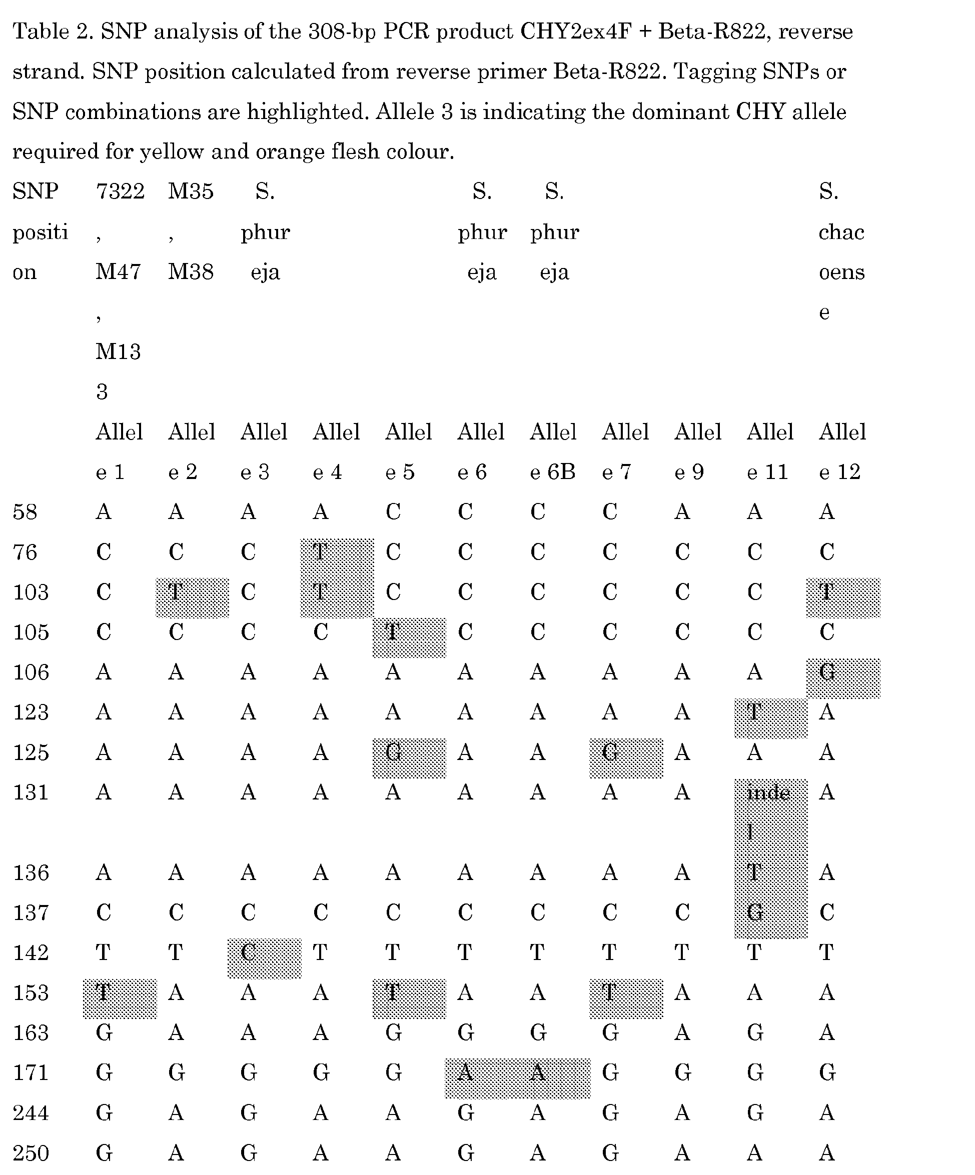

A correlation between presence of allele 3 and yellow flesh colour was observed in a number of diploids. Furthermore, it was observed that a tagging SNP (SNP 142C) distinguished CHY2 allele 3 from all other alleles. Allele 3 is identical to the dominant allele B described by Brown *et al.* (2006), because sequencing of allele 3 showed the presence of allele B-specific primer sequence YellowF1. This allele is considered to be the dominant Y allele at the *Yellow* (*Y*) locus.

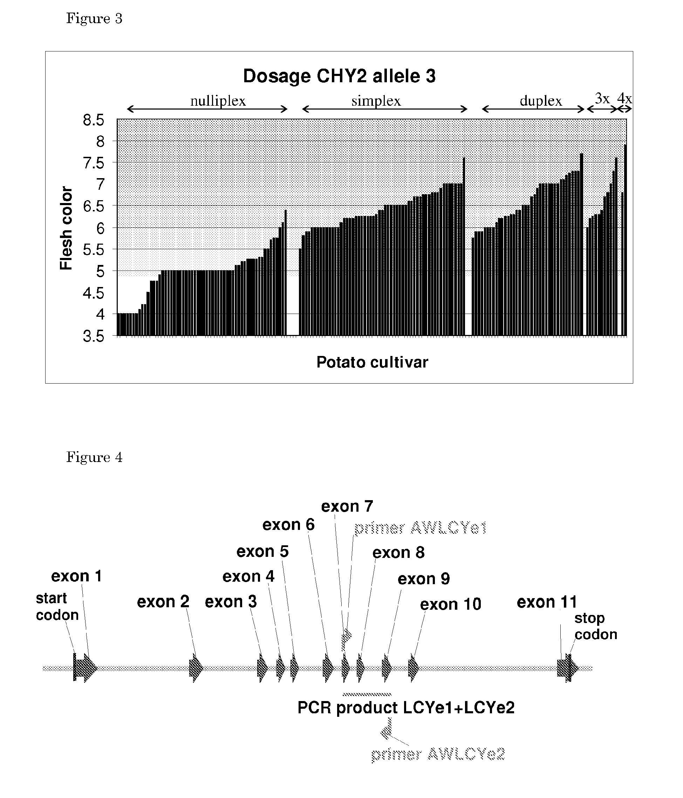

Next, a PCR analysis was performed on the DNA of a set of 225 tetraploid potato cultivars. The same PCR fragment that was analysed for the monoploids and diploids was amplified in the tetraploids. Direct sequencing was performed, which resulted in the discovery of three additional alleles (Table 2; alleles 7, 9, 11). The dosage of SNP 142C (i.e. allele 3) was determined from the trace files. Dosage of allele 3 could also be estimated by using a CAPS marker (Figure 2). The allele 3 dosage was related to the flesh colour value, see Table 3. A flesh colour value of 5.5 or lower represents white flesh, whereas a flesh colour value higher than 5.5 indicates yellow flesh. As is shown in Table 3 presence or absence of CHY2 allele 3 is correlated with flesh colour: the group of cultivars lacking allele 3 have a mean value of 5.1 (white flesh), whereas the cultivars simplex, duplex, triplex or quadruplex for allele 3 have a mean value higher than 6 (yellow flesh). The data suggest a dosage effect of allele 3 as the quadruplex genotypes have the highest mean value. However, this group consists of only two genotypes. Although there is a clear correlation between presence of CHY2 allele 3 and yellow flesh, the intensity of the yellow flesh colour shows considerable variation within the different dosage groups, as shown in Figure 3. This was also observed by Brown *et al.* (2006).

**Table 3. Mean value for tuber flesh colour for tetraploid S. tuberosum genotypes with 0, 1, 2, 3 or 4 copies of the CHY2 allele 3 (SNP142C).**

| CHY2 allele 3 dosage | Mean tuber flesh colour value | Flesh colour | Number of genotypes |
|---|---|---|---|
| 0x (nulliplex) | 5.1 | White | 69 |
| 1x (simplex) | 6.3 | Yellow | 71 |
| 2x (duplex) | 6.6 | Yellow | 46 |
| 3x (triplex) | 6.4 | Yellow | 10 |
| 4x (quadruplex) | 7.3 | Yellow | 3 |

To evaluate if CHY2 alleles other than allele 3 have a (small) influence on flesh colour the CHY2 allele composition was determined for 199 of the 225 tetraploid potato genotypes by analyzing tagging SNPs. Large differences in allele frequency were observed. Four major alleles were observed: alleles 5 (35%), 3 (26%), 1 (20%) and 2 (13%). Four minor alleles were observed: alleles 6 (4%), 11 (2%), 7 (0.8%) and 9 (0.3%). Minor allele 6 has been present in the *Solanum tuberosum* gene pool for a long time, as it is observed in cultivar Jaune d'Or (from before 1850), Paterson's Victoria (1856), Magnum Bonum (1876), Irish Cobbler (1876), Duke of York (1891), British Queen (1894), Eigenheimer (1895), Epicure (1897) and Irish Queen (before 1900). Minor alleles 11, 7 and 9 seem to be new introductions in the *Solanum tuberosum* gene pool. Allele 11, containing an indel (1-nt deletion) in the analyzed PCR fragment, is present in a number of cultivars derived from VTN 62-33-3 (1962), suggesting that this might be an *S. vernei* allele (see Kort *et al.* 1972).

None of the alleles 1, 2, 5, 6, 7, 9 or 11 were related to yellow flesh colour. Furthermore, none of these alleles influenced flesh colour value within the white/creamy flesh colour class, nor within the yellow flesh colour class. Thus, the variation in intensity of yellow flesh colour can not be explained by the composition of the other CHY2 alleles in the simplex, duplex and triplex allele 3 groups. Therefore, it was suspected that other genes involved in the carotenoid biosynthetis pathway influenced the intensity of the yellow flesh colour.

### SNP analysis of the lycopene epsilon cyclase gene (LCYe)

The lycopene epsilon cyclase gene product is required for the synthesis of α-carotene, the precursor of lutein (see Tanaka *et al.* 2008). Silencing of the LCYe gene resulted in a significant increase in the beta-carotenoids (Diretto *et al.* 2006). An increase in the level of beta-carotene and zeaxanthin is expected to result in a darker yellow flesh.

To be able to analyse allelic variation for the LCYe gene in potato we first needed information on the genomic sequence of the LCYe gene. First, by BLASTN search a tomato BAC sequence (AC216345, from clone LE_HBa-11D12, from chromosome 12) was found containing the tomato homologue of LCYe. Later, a potato BAC sequence (BAC RH091F11-2, see Potato Genome Sequencing Consortium (PGSC)) was observed to contain the potato homologue (Figure 4). Primers AWLCYe1 and AWLCYe2 were designed, which amplified a fragment spanning exon 7, intron 7, exon 8, intron 8 and exon 9 of the LCYe genomic sequence. PCR fragments from the monoploids and diploids were directly sequenced, and at least 5 different alleles could be observed (Table 4). Potato BAC RH091F11-2 proved to contain allele 4, whereas two other BACs (RH196E12-4 and RH132J19-7) contained the other allele (allele 1) of diploid genotype RH89-039-16.

Two alleles (allele 2 and allele 5) contain a T at SNP position 545.
This nucleotide causes an amino acid change S401F. This amino acid change is not tolerated according to the SIFT program (Sorting Intolerant From Tolerant, Ng and Henikoff 2006), and possibly damaging according to the PolyPhen program (Polymorphism Phenotyping). Diploid genotype C contains LCYe alleles 2 and 3, while genotype E contains LCYe alleles 1 and 2. Thus, C and E have LCYe allele 2 in common. Therefore, 25% of the progeny of a cross between these genotypes is expected to be homozygous for allele 2.

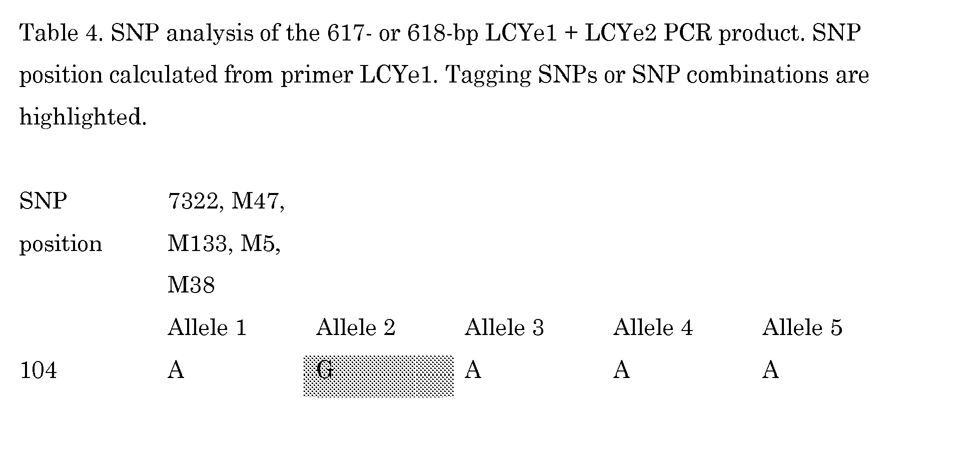

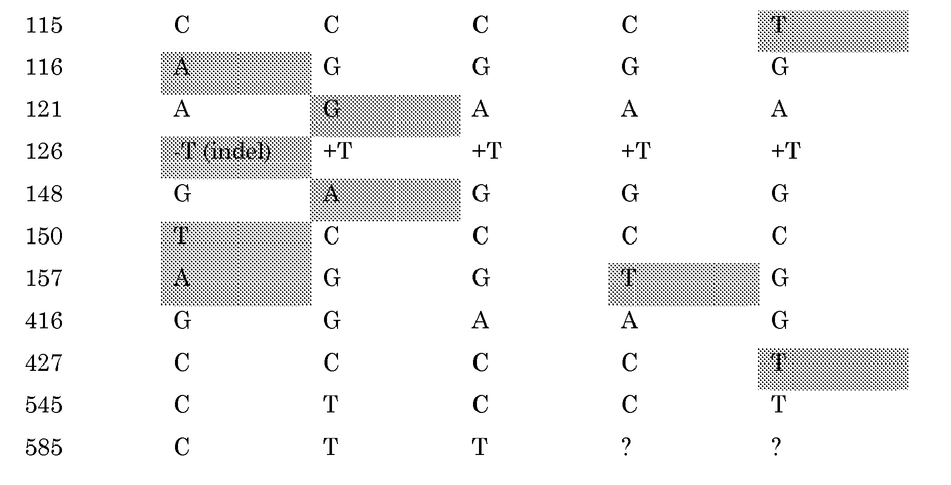

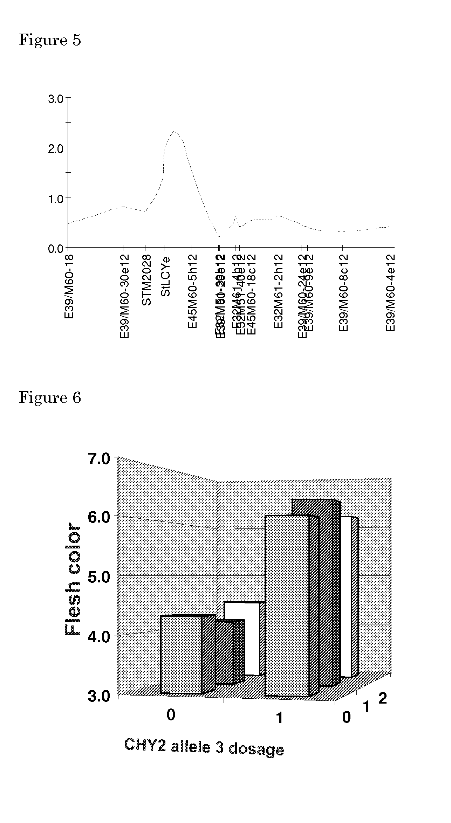

Some of the progeny of the CxE cross have a much higher flesh colour value than the yellow parent C, i.e. some have orange flesh. To investigate whether homozygosity of LCYe allele 2 leads to a difference in flesh colour, 94 CxE progeny plants were analyzed for their LCYe allele composition. Four different allelic compositions were expected: 1+2, 1+3, 2+2 and 2+3. To distinguish the different alleles CAPS markers were developed. The AWLCYe1+AWLCYe2 PCR product was digested with *Ssi*I, which distinguished allele 2 from alleles 1 and 3. By digesting the AWLCYe1+AWLCYe2 PCR product with *Hpy*CH4IV allele 1 could be distinguished from alleles 2 and 3. By combining the results of these two CAPS markers the LCYe allele composition of each CE progeny could be determined. Data were used to localize the LCYe gene on the CxE linkage map. LCYe mapped to a position on chromosome 12, as expected, close to the STM2028 microsatellite marker and the SUS4 gene at the Southern distal end of the chromosome (Figure 5).

Dosage of LCYe allele 2 and dosage of CHY2 allele 3 were plotted against flesh colour value (Figure 6). As shown in this figure there is a strong correlation between presence of CHY2 allele 3 and a high value for flesh colour, whereas dosage of LCYe allele 2 does not seem to have an influence on flesh colour. This suggests that a different gene than LCYe must have an influence on intensity of yellow flesh colour.

### SNP analysis of the zeaxanthin epoxidase gene (ZEP)

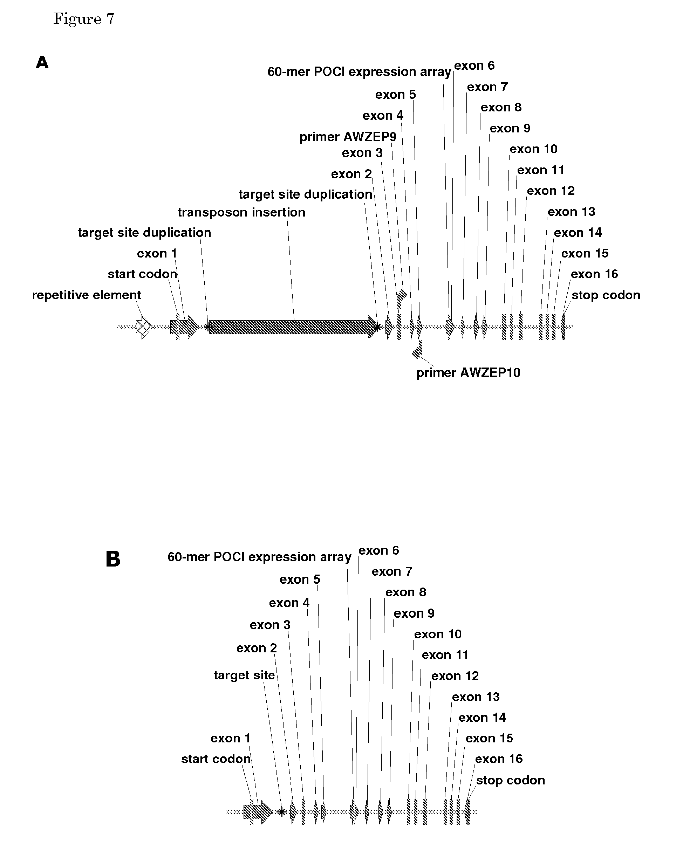

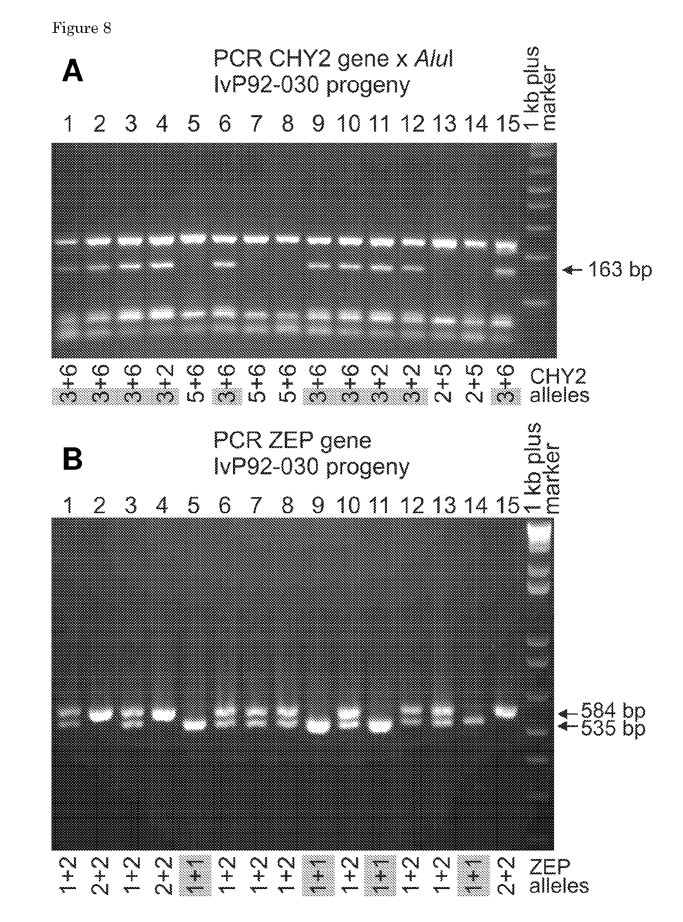

As mentioned above, another candidate gene for orange tuber flesh in potato is the ZEP gene. A genomic sequence of the tomato ZEP gene was found to be present in BAC C02HBa0104A12.1. This BAC was anchored to tomato chromosome 2, which is in agreement with the map position of the pepper *(Capsicum)* ZEP gene on chromosome 2 (Thorup *et al.* 2000). Using the tomato BAC sequence and potato ZEP cDNA sequence DQ206629 primers were designed allowing amplification and sequencing of the complete potato ZEP gene (Figure 7). Using primer combination AWZEP9 + AWZEP10 five different alleles could be distinguished in the monoploid and diploid *S. tuberosum* genotypes (Table 5, alleles 1-5). SNP analysis provided multiple SNPs that can be used as markers to discriminate ZEP allele 1 from the other ZEP alleles. These SNPs are G83A, T99G, A113T, A154G, G165C, A180G, T209C, T231A, T375G, A384T, A389C, A415G, C422-, C422T, T426- and T426C. Furthermore, the AWZEP9 + AWZEP10 PCR product, spanning exon 3, intron 3, exon 4, intron 4 and exon 5, showed the presence of a relatively large indel in intron 4 of ZEP allele 1. Therefore, ZEP allele 1 misses a sequence of 49 bp, which is present in alleles 2, 3, 4 and 5. Thus, ZEP allele 1 could be distinguished from the other alleles based on the presence of the 49 bp-indel as detected by gel electroforesis. The AWZEP9+AWZEP10 PCR product of allele 1 is 535 bp long, whereas this PCR product is 584 bp for the other alleles (Figure 8B).

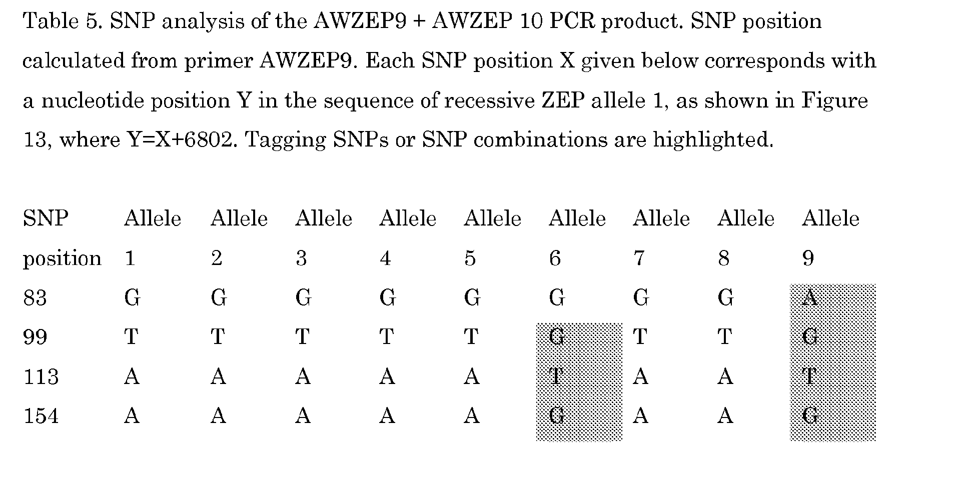

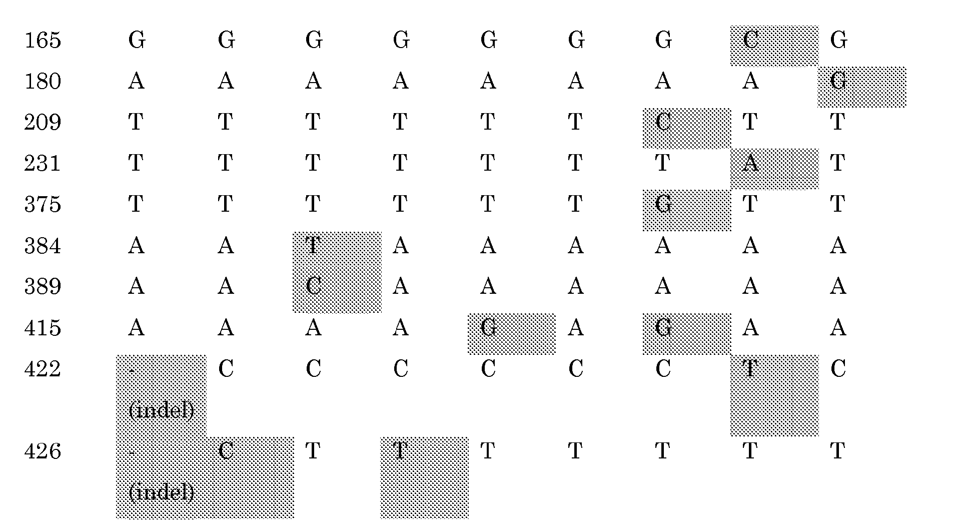

ZEP allele composition was determined in diploid orange-fleshed genotypes Papa Pura, Andean Sunrise, Yema de Huevo, IvP92-030-11 and IvP01-84-19. All five genotypes proved to be homozygous for ZEP allele 1. As these five genotypes are not closely related to each other this suggests the involvement of ZEP allele 1 in the orange flesh phenotype.

Genetic evidence for the involvement of ZEP allele 1 in orange flesh colour was obtained from cosegregation in the diploid IvP92-030 population (progeny of the cross between diploids G254 and SUH2293). This population was analyzed for CHY2 and ZEP allele compositions (Figure 8). Parent G254 contained CHY2 alleles 2 and 6, while parent SUH2293 contained CHY2 alleles 3 and 5. Progeny with allele combinations 2+3, 2+5, 3+6 and 5+6 were all observed. Both parents G254 and SUH 2293 contained ZEP alleles 1 and 2. Progeny with allele combinations 1+1, 1+2 and 2+2 were obtained in numbers compatible with the expected 1:2:1 ratio. Only progeny plants IvP92-030-9 and IvP92-030-11, containing CHY2 allele 3 and homozygous for ZEP allele 1, showed the orange-fleshed phenotype. This suggests a model in which there is a requirement for the presence of dominant CHY2 allele 3 and homozygosity for recessive ZEP allele 1 to obtain an orange-fleshed potato.

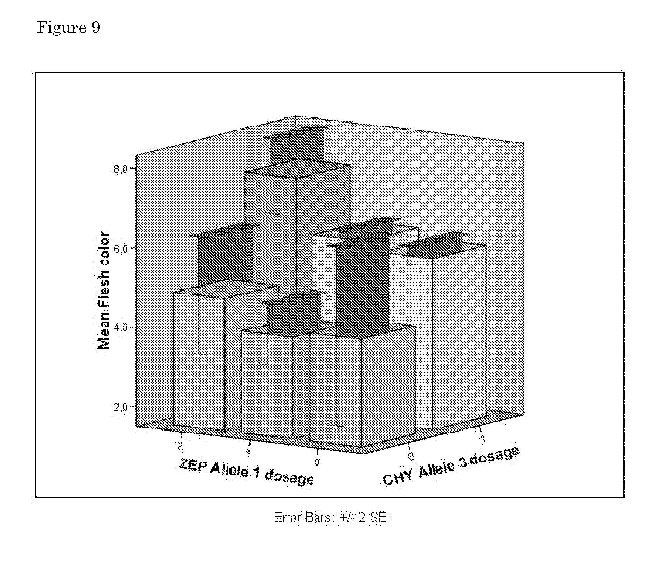

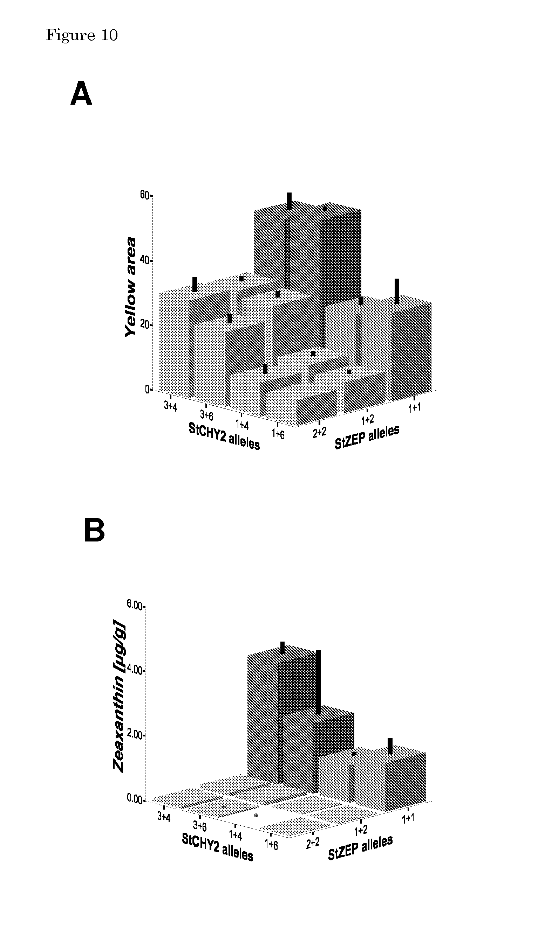

To investigate this further progeny of the CxE cross was analyzed for ZEP allele composition. Both C and E parents contain ZEP alleles 1 and 2. A CAPS marker was developed to easily distinguish both ZEP alleles. For this, PCR product AWZEP9 + AWZEP10 was digested with the enzyme *Hin*6I*.* The PCR product of ZEP allele 1 remained undigested, whereas the PCR product of allele 2 was digested into fragments of 439 and 145 bp. 94 CxE progeny was analyzed with this CAPS marker. The ZEP gene was mapped in the CxE population on chromosome 2 in a similar position as the one on tomato chromosome 2. Dosage of ZEP allele 1 and dosage of CHY2 allele 3 were related to flesh colour value (Figure 9, 10A). These figures show that homozygosity of ZEP allele 1 in combination with presence of CHY2 allele 3 results in a significantly higher mean flesh colour value. A QTL analysis for "yellow area" in the CxE population resulted in a highly significant QTL on chromosome 2, on the same position as the ZEP gene (B. Kloosterman, unpublished results).

### Influence of ZEP allele 1 on zeaxanthin levels.

For a number of CxE progeny the amounts of individual carotenoids were determined (Tables 6 and 7). A small number of progeny proved to contain increased levels of zeaxanthin. These progeny invariably were homozygous for ZEP allele 1. In Figure 10B the relation between CHY2 allele composition, ZEP allele composition and zeaxanthin contents is displayed. This figure demonstrates that zeaxanthin accumulates in considerable amounts in genotypes homozygous for ZEP allele 1. When dominant CHY2 allele 3 is also present the increase in zeaxanthin levels is even higher. These results suggest that orange flesh colour indicates the presence of a zeaxanthin level of more than 250 µg/ 100g fresh weight tuber.

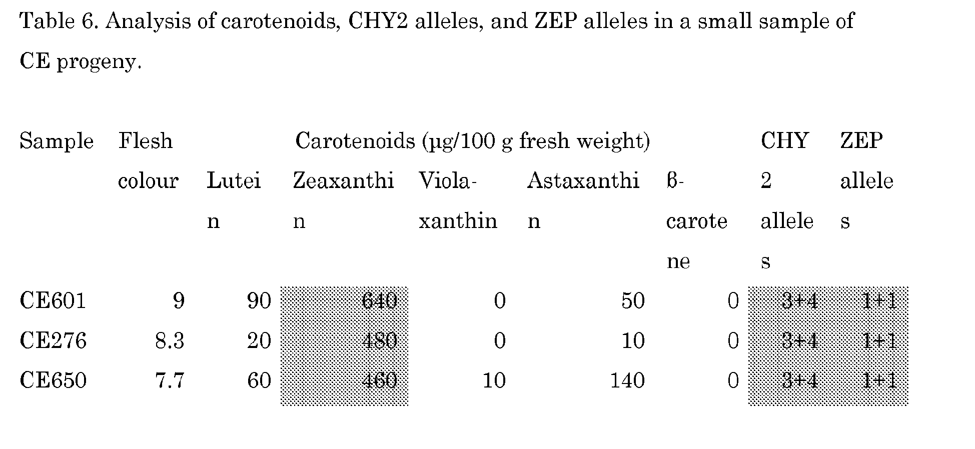

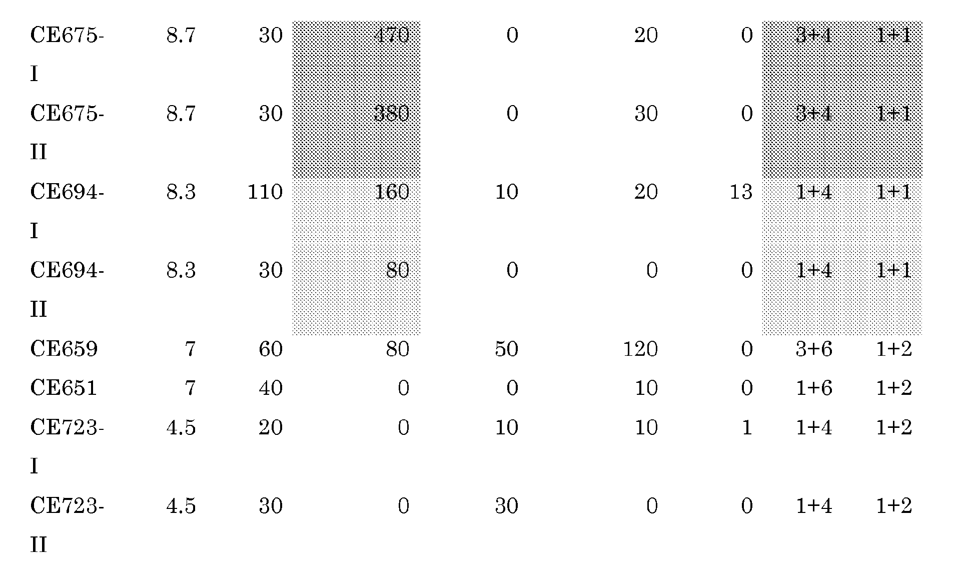

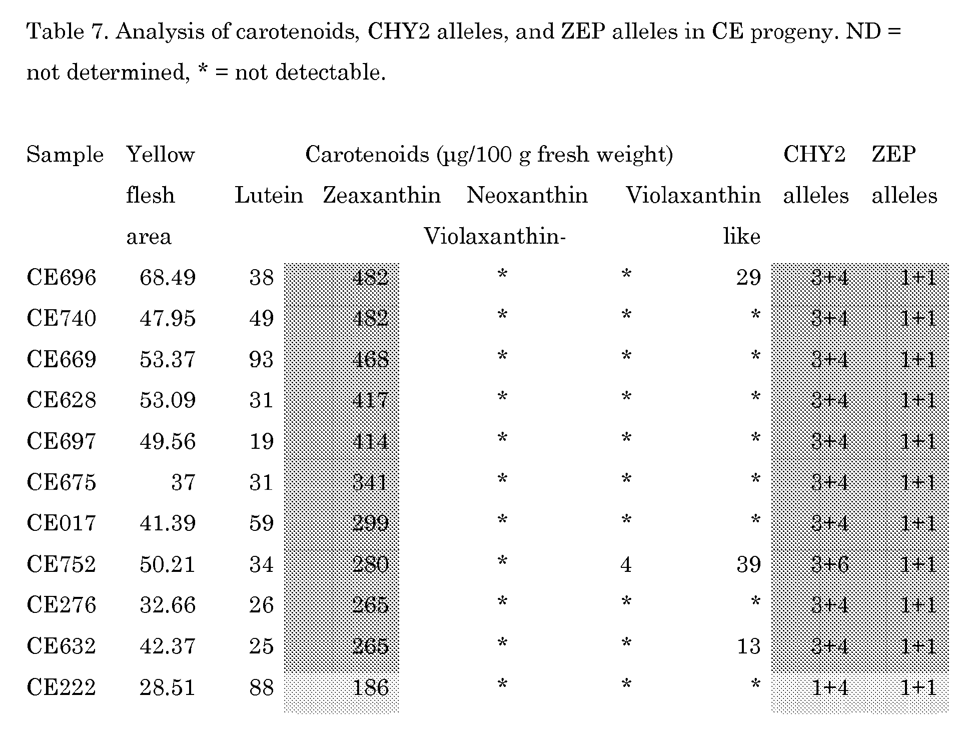

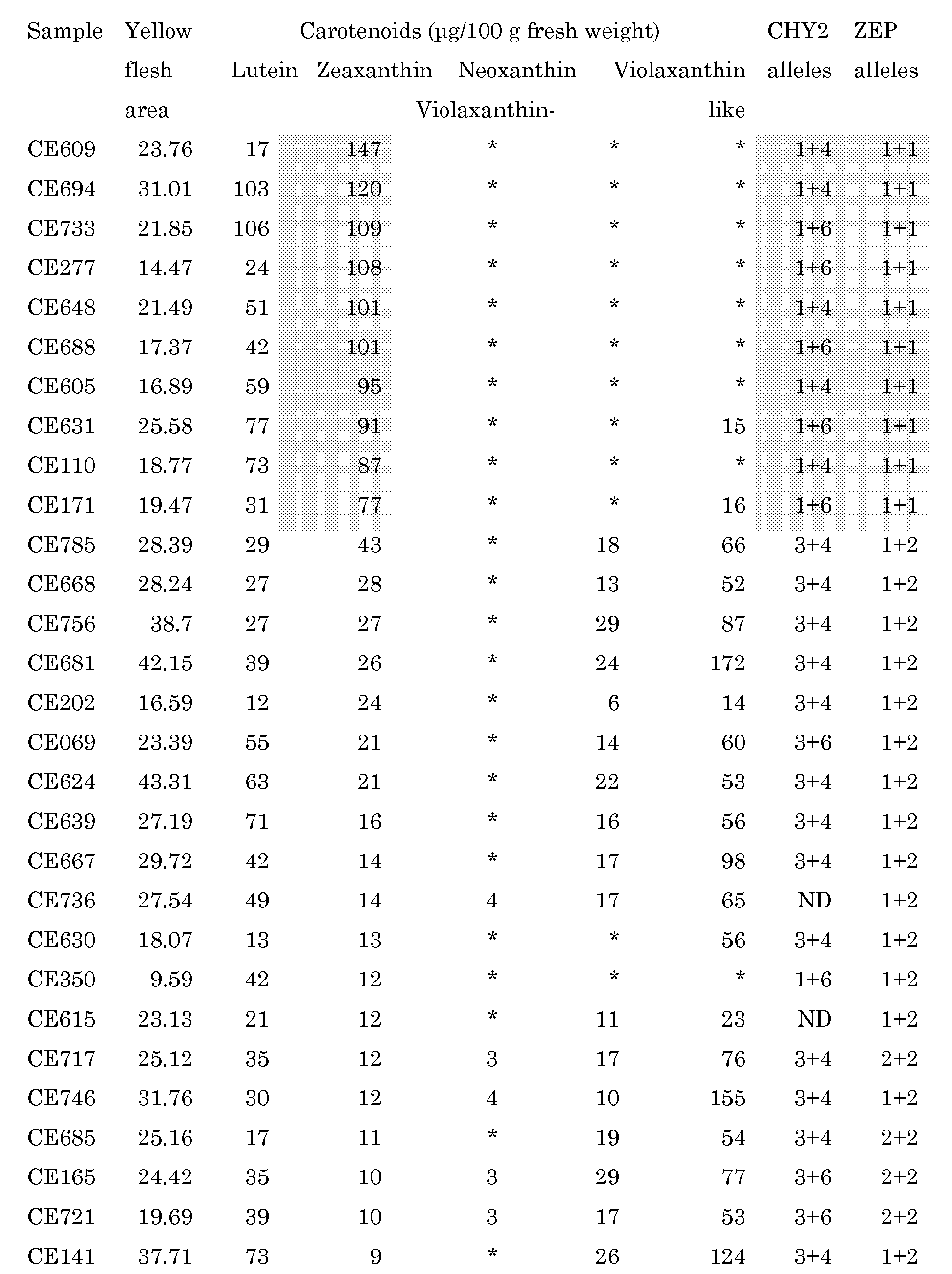

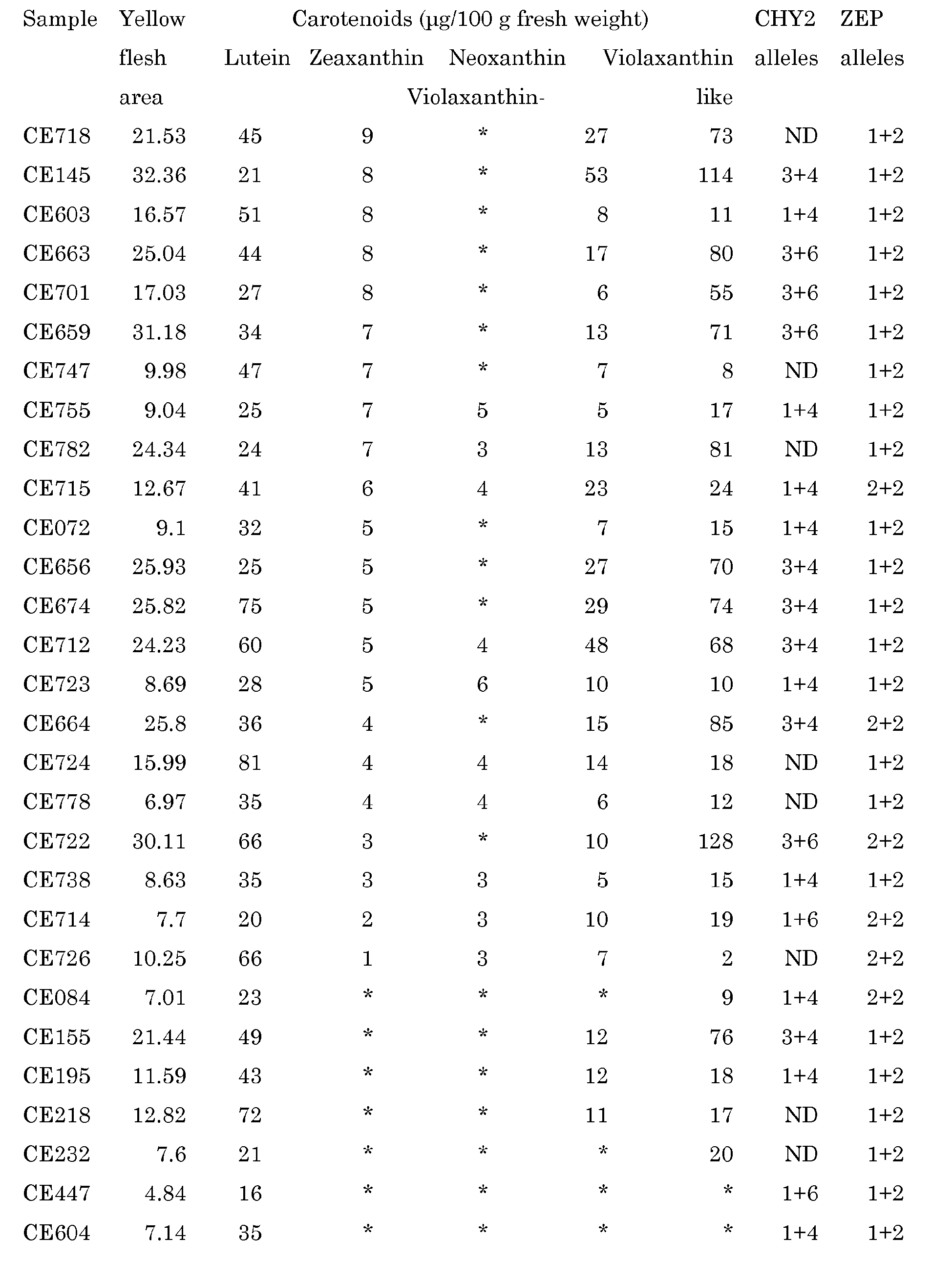

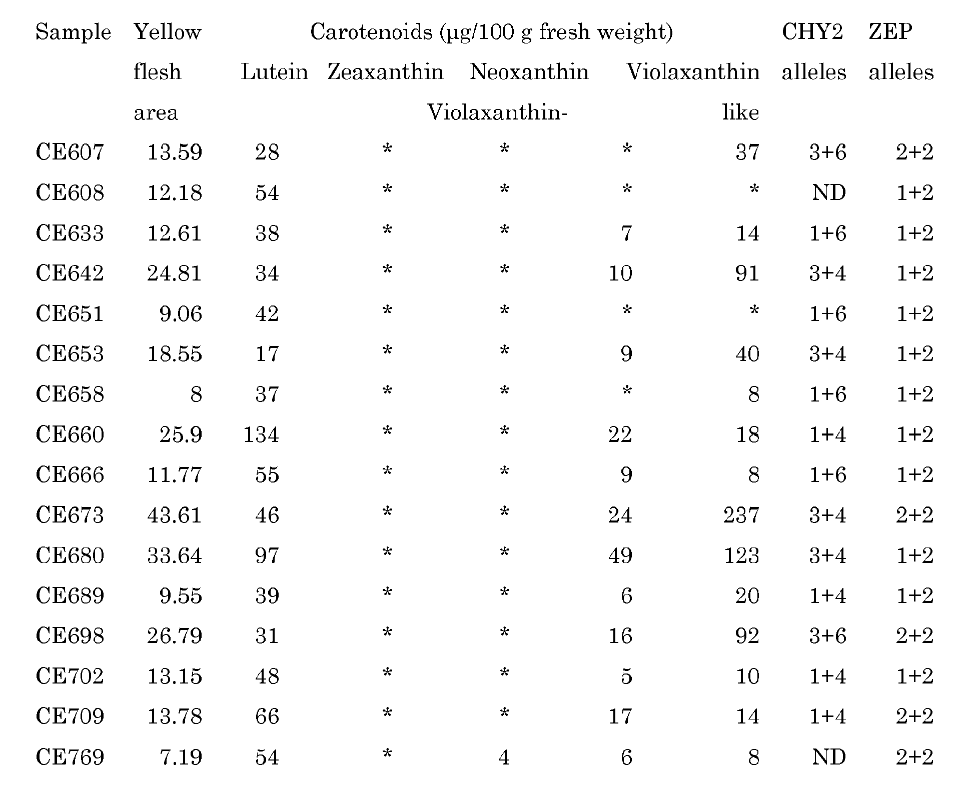

### Recessive inheritance of orange tuber flesh.

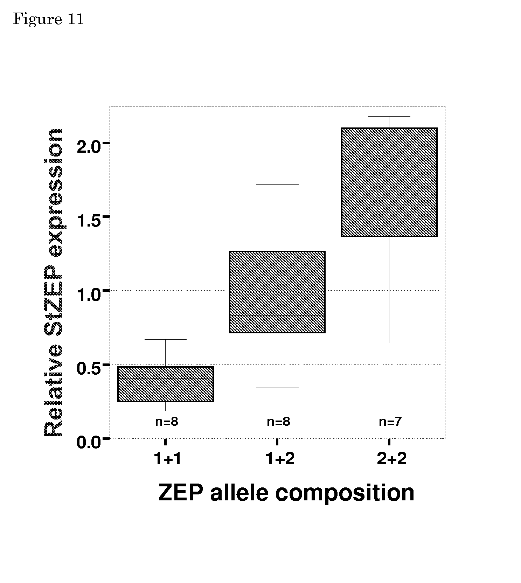

Expression analysis using the 44k POCI array indicated that tuber RNA from parents C and E, both heterozygous for alleles 1 and 2, showed a similar level of hybridization (B. Kloosterman, unpublished results). Tuber RNA from CxE progeny homozygous for ZEP allele 2 showed a higher level of hybridization with the ZEP 60-mer than both parents, whereas tuber RNA from CxE progeny homozygous for ZEP allele 1 showed a lower level of hybridization than both parents. This may reflect a difference in homology of the ZEP alleles with the 60-mer. It was found that the 60-mer on the POCI array (Kloosterman *et al.* 2008) is identical to a sequence in exon 6 of ZEP allele 1, while there is one mismatch with the sequence in ZEP allele 2. If the mismatch would result in a lower level of hybridization it would be expected that RNA from progeny homozygous for ZEP allele 2 would show a lower level of hybridization. However, the opposite was observed. Therefore, the array results suggest that ZEP allele 2 is expressed at a higher level than ZEP allele 1. This was confirmed by quantitative RT-PCR (Figure 11). Because of this lower expression level ZEP allele 1 can be considered to be a recessive allele. A similar observation was made by Morris *et al.* (2004) who found that high carotenoid-accumulating *S. phureja* DB375\1 (later renamed cultivar Inca Dawn) showed low expression of the ZEP gene. They observed an inverse relationship between zeaxanthin transcript level and total carotenoid content in a range of potato germplasm.

To investigate the reason for the lower expression level of ZEP allele 1 a 1.8-kb fragment containing the promoter of this allele was obtained by Genome Walking and was sequenced. A BLASTN analysis revealed that the 5'part of this sequence contained a repetitive element (present on several chromosomes of *S. tuberosum* and *S. lycopersicum).* An analysis of cis-regulatory elements showed that the ZEP promoter contains light-regulated, phytochrome-regulated, and water stress-regulated boxes, as well as hypo-osmolarity responsive and sugar-repression elements. Subsequently, this sequence was compared with the promoter sequence of ZEP allele 2 (as present in monoploid M133), see Figure 12. Although a number of SNPs was observed, no obvious differences were found that could explain the different expression levels.

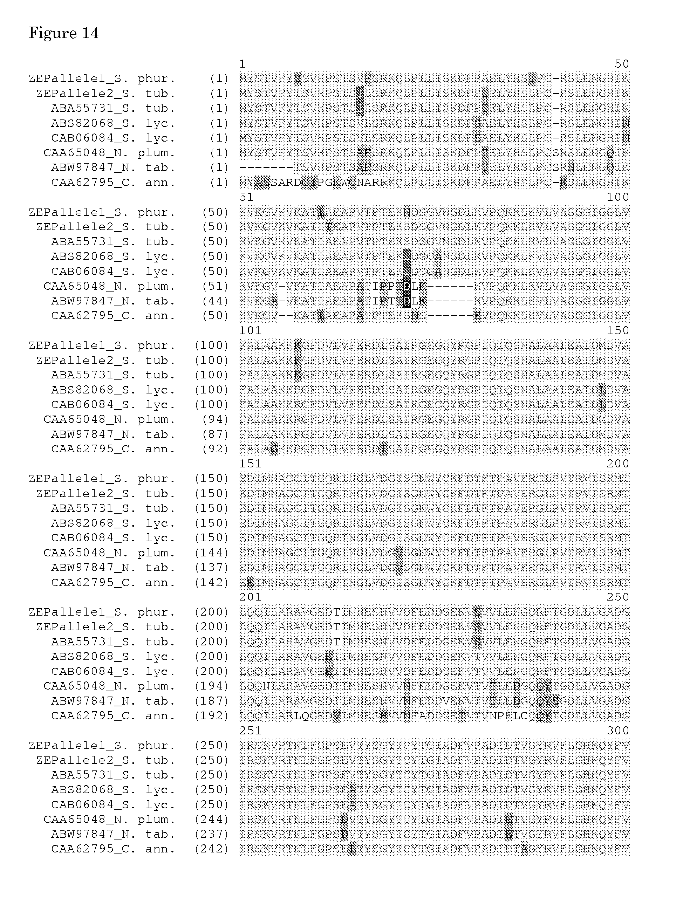

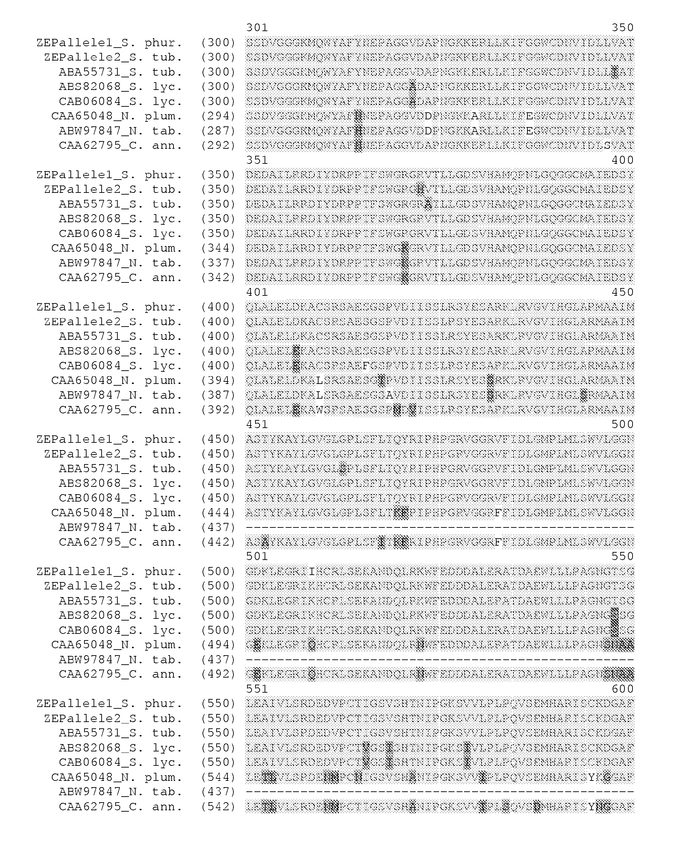

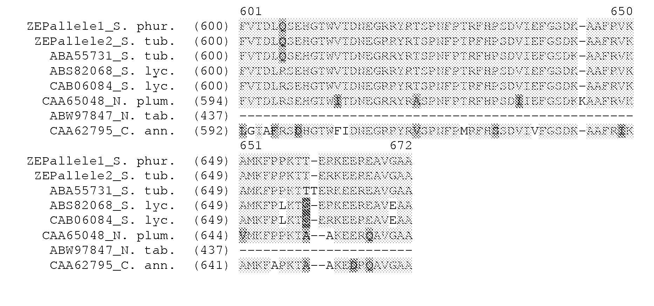

Next, the complete genomic sequence, including all exons and introns, was determined for both ZEP alleles 1 and 2 (Figure 7 and Figure 12). The exon sequences were translated into protein sequences and aligned to each other (Figure 13). Although a number of amino acid changes were observed, especially in the first exon, no obvious amino acid change was found predicting a non-functioning enzyme according to SIFT. Alignment of the deduced amino acid sequences of ZEP alleles 1 and 2 with ZEP protein sequences of other Solanaceous species (Figure 14) indicated that the differences in amino acids between ZEP alleles 1 and 2 mostly occurred in less conserved regions.

However, a large difference in size was observed in the first intron. Intron 1 in ZEP allele 2 is 389 bp in size, comparable with the 438-bp intron 1 of the tomato ZEP genomic sequence. However, the size of intron 1 in ZEP allele 1 is 4509 bp. By comparing the sequences of the first intron of ZEP alleles 1 and 2 we observed that a 4102-bp transposon-like sequence had integrated in the sequence of intron 1 as present in allele 2, causing a target site duplication of 18 bp.

### Occurrence of ZEP allele 1 in the tetraploid potato genepool

To determine the frequency of ZEP allele 1 in the tetraploid potato genepool, PCR with primers AWZEP9 + AWZEP10 was performed using DNA from a set of 221 tetraploid potato cultivars (D'hoop *et al.* 2008) and some additional cultivars. From 230 genotypes that yielded a PCR product only 5 contained ZEP allele 1, all in simplex. These were genotypes Black 1256, Prevalent (descendent from Black 1256), Producent (descendent from Prevalent), Lady Claire and Pallas (both descendents from *S. phureja* PHUR 71-464-7). This indicates that ZEP allele 1 is a rare allele in the tetraploid potato gene pool (frequency 0.5%).

ZEP allele composition was determined in 111 tetraploid potato genotypes not containing ZEP allele 1, by sequencing the AWZEP9+AWZEP10 PCR product. Four additional alleles were observed besides the alleles present in the monoploid and diploid genotypes (Table 4). Alleles 2 (36%), 3 (25%), 4 (14%) and 5 (20%) were major alleles, whereas alleles 6 (0.9%), 7 (0.5%), 8 (1.6%) and 9 (1.6 %) were minor alleles. The minor alleles are all recent introductions in the *S. tuberosum* gene pool. Alleles 3 to 9 had an intron 1 of similar size as intron 1 in allele 2. Therefore none of these alleles contained the transposon insertion present in allele 1.

### Discussion

It is concluded that homozygosity for ZEP allele 1 in the presence of dominant CHY2 allele 3 is causing the orange flesh colour phenotype, due to high levels of zeaxanthin. Furthermore, it is concluded that ZEP allele 1 is a recessive allele. The accumulation of zeaxanthin is not caused by impaired function of the ZEP protein resulting from amino acid changes, as we observed no obvious amino acid changes in allele 1 compared with allele 2. Rather, the recessiveness is caused by a lower steady state mRNA level, as determined by qRT-PCR.

Morris *et al.* (2004) observed an inverse relationship between the ZEP transcript level and the total tuber carotenoid content. They investigated transcript level by quantitative RT-PCR using primers designed on the basis of tomato ZEP cDNA sequence Z83835. However, the forward primer contains an A at position 18 instead of G, as present in potato ZEP cDNA DQ206629 and potato EST CK278242. Only G was observed at this position in our ZEP alleles, including allele 1. This indicates a difference in ZEP sequences between tomato and potato. As this SNP position is close to the 3' end of the forward primer this mismatch may have a considerable influence on overall level of amplification in the RT-PCR experiment.

qRT-PCR was performed on a diploid population segregating for ZEP alleles 1 and 2, using primers without mismatches. A clear difference in expression levels was observed between genotypes homozygous for allele 1, heterozygous (allele 1+2) and homozygous for allele 2.

A small number of SNPs was observed in the promoter sequence of allele 1 compared with the sequence of allele 2, which may explain the difference in expression level between the two alleles. However, it is more likely that the difference in expression level is caused by the large transposon insertion in the first intron of allele 1. ESTs have been observed that show homology to this transposon sequence in the potato EST databases, probably derived from copies of this transposon elsewhere in the potato genome. This large insertion may cause inefficient splicing of the premRNA into mature mRNA, or alternative splicing caused by cryptic splice sites. Hanson (1989) reported that efficient intron splicing in plants may be constrained by intron length. Ohmori *et al.* (2008) reported that integration of a transposon in intron 4 of rice gene DL resulted in reduced expression of the gene. A lower expression level of the zeaxanthin epoxidase gene results in the accumulation of zeaxanthin, at the expense of antheraxanthin, violaxanthin and neoxanthin (Tanaka *et al.* 2008; see Table 7). As zeaxanthin is relatively orange coloured, while antheraxanthin, violaxanthin and neoxanthin are (light) yellow, this explains the orange flesh phenotype of the potato genotypes homozygous for ZEP allele 1 (and containing CHY2 allele 3).

ZEP allele 1 probably is an *S. phureja* ZEP allele, as it is almost absent in the *S. tuberosum* gene pool, and only present in a few genotypes with *S. phureja* in their ancestry.

### Conclusions

Genetic analyses of the allelic composition of multiple potato cultivars in association with the tuber flesh phenotype resulted in the identification of several unique features that are indicative for a recessive ZEP allele. These unique features provide diagnostic capacity to distinguish the ZEP allele that is relevant for increasing zeaxanthin levels in a plant line. The features can therefore be applied in methods for increasing zeaxanthin levels in a plant line, comprising crossing the plant line and marker-assisted selection of suitable plants.

By using the methods according to the invention, marker-assisted selection of parents for well designed crossing purposes can now be performed at DNA level. Furthermore, marker-assisted selection of the desirable progeny can be performed as early as seedling stage. The methods according to the invention also allow to select "hidden carriers" with an elevated number of at most three alleles of the recessive ZEP allele1, although such plants do not display any phenotype. Thus, the method for increasing the zeaxanthin level in a plant line according to the invention enables the design of rational breeding strategies, without being hampered by the complexities of breeding a recessively and epistatically inherited trait.

Furthermore, the method for increasing the zeaxanthin level in a plant line according to the invention is not only much cheaper but also saves the growing season that would otherwise be needed for obtaining analysable tubers.

### REFERENCES

Andre CM, Oufir M, Guignard C, Hoffmann L, Hausman JF, Evers D, Larondelle Y (2007) Antioxidant profiling of native Andean potato tubers (Solanum tuberosum L.) reveals cultivars with high levels of β-carotene, α-tocopherol, chlorogenic acid, and petanin. J Agric Food Chem 55: 10839-10849
Bachem CWB, Oomen RJFJ, Visser RGF (1998) Transcript imaging with cDNA-AFLP: A step-by-step protocol. Plant Mol Biol Rep 16: 157-173
Binding H, Nehls R, Schieder O, Sopory SK, Wenzel G (1978) Regeneration of mesophyll protoplasts isolated from dihaploid clones of Solanum tubersoum. Physiol Plant 43: 52-54
Bino RJ, de Vos CHR, Lieberman M, Hall RD, Bovy A, Jonker HH, Tikunov Y, Lommen A, Moco S, Levin I (2005) The light-hyperresponsive high pigment-2dg mutation of tomato: alterations in the fruit metabolome. New Phytologist 166: 427-438
Bonierbale MW, Plaisted RL, Tanksley SD (1988) RFLP maps based on a common set of clones reveal modes of chromosomal evolution in potato and tomato. Genetics 120: 1095-1103
Bradshaw JE, Ramsay G (2005) Utilisation of the Commonwealth Potato Collection in potato breeding. Euphytica 146: 9-19
Breithaupt DE, Bamedi A (2002) Carotenoids and carotenoid esters in potatoes (Solanum tuberosum L.): new insights into an ancient vegetable. J Agric Food Chem 50: 7171-7181
Bremer G (1961) Problems in breeding and cytology of sugar cane. IV. The origin of the increase of chromosome number in species hybrids of saccharum. Euphytica 10: 325-342.
Brown CR (2008) Breeding for phytonutrient enhancement of potato. Am J Pot Res 85: 298-307
Brown CR, Edwards CG, Yang CP, Dean BB (1993) Orange flesh trait in potato: Inheritance and carotenoid content. J Amer Soc Hort Sci 118: 145-150
Brown CR, Kim TS, Ganga Z, Haynes K, De Jong D, Jahn M, Paran I, De Jong W (2006) Segregation of total carotenoid in high level potato germplasm and its relationship to beta-carotene hydroxylase polymorphism. Am J Potato Res 83: 365-372
Brown CR, Culley D, Bonierbale M, Amorós W (2007) Anthocyanin, carotenoid content, and antioxidant values in native South American potato cultivars. HortScience 42: 1733-1736
Burgos G, Salas E, Amoros W, Auqui M, Muñoa L, Kimura M, Bonierbale M (2009) Total and individual carotenoid profiles in the Phureja group of cultivated potatoes: I. Concentrations and relationships as determined by spectrophotometry and HPLC. J Food Compos Anal (2009) in press
Celis-Gamboa BC (2002) Life cycle of the potato (Solanum tuberosum L.): from crop physiology to genetics. Ph.D. thesis, Wageningen University, Wageningen, The Netherlands
Chen CH, Goeden-Kallemeyn YC (1979) In vitro induction of tetraploid plants from colchicine-treated diploid daylily callus. Euphytica 28(3): 705-709
De Vries SE, Ferwerda MA, Loonen AEHM, Pijnacker LP, Feenstra WJ (1987) Chromosomes in somatic hybrids between Nicotiana plumbaginifolia and a monoploid potato. Theor Appl Genet 75: 170-176
De Vries-Uijtewaal E, Gilissen LJW, Flipse E, Sree Ramulu K, Stiekema WJ, de Groot B (1989) Fate of introduced genetic markers in transformed root clones and regenerated plants of monohaploid and diploid potato genotypes. Theor Appl Genet 78: 185-193
D'hoop BB, Paulo MJ, Mank RA, van Eck HJ, van Eeuwijk FA (2008) Association mapping of quality traits in potato (Solanum tuberosum L.). Euphytica 161: 47-60
D'hoop BB (2009) Association mapping in tetraploid potato. PhD thesis Wageningen University, The Netherlands
Diretto G, Tavazza R, Welsch R, Pizzichini D, Mourgues V, Beyer P, Giuliano G (2006) Metabolic engineering of potato tuber carotenoids through tuber-specific silencing of lycopene epsilon cyclase. BMC Plant Biology 6:13
Francis A, Jones RN (1989) Heritable nature of colchicine induced variation in diploid Lolium perenne. Heredity 62: 407-410
Fruwirth C (1912) Zur Züchtung der Kartoffel. Deutsche Landwirtschaftliche Presse 39: 551-552, 565-567
Handelman GJ, Dratz EA, Reay CC, van Kuijk FJGM (1988) Carotenoids in the human macula and whole retina. Invest Ophthalmol Vis Sci 29: 850-855
Hanneman Jr RE, Peloquin SJ (1967) Crossability of 24-chromosome potato hybrids with 48-chromosome cultivars. Eur Potato J 10: 62-73
Hanson MR (1989) Tracking down plant genes: paths, patterns and footprints. Plant Cell 1: 169-172
Hoogkamp TJH, van den Ende RGT, Jacobsen E, Visser RGF (2000) Development of amylose-free (amf) monoploid potatoes as new basic material for mutation breeding in vitro. Potato Res 43: 179-189
Hovenkamp-Hermelink JHM, Jacobsen E, Pijnacker LP, de Vries JN, Witholt B, Feenstra WJ (1988) Cytological studies on adventitious shoots and minitubers of a monoploid potato clone. Euphytica 39: 213-219
Iwanzik W, Tevini M, Stute R, Hilbert R (1983) Caotenoidgehalt und - zusammensetzung verschiedener deutscher Kartoffelsorten und deren Bedeutung für die Fleischfarbe der Knolle. Potato Res 26: 149-162
Jacobs JME, van Eck HJ, Arens P, Verkerk-Bakker B, te Lintel Hekkert B, Bastiaanssen HJM, El-Kharbotly A, Pereira A, Jacobsen E, Stiekema WJ (1995) A genetic map of potato (Solanum tuberosum) integrating molecular markers, including transposons, and classical markers. Theor Appl Genet 91: 289-300
Jacobsen E (1980) Increase of diplandroid formation and seed set in 4x X 2x crosses in potatoes by genetical manipulation of dihaploids and some theoretical consequences. Z Pflanzenzuecht 85: 110-121
Jacobsen E, Hovenkamp-Hermelink JHM, Krijgsheld HT, Nijdam H, Pijnacker LP, Witholt B, Feenstra WJ (1989) Phenotypic and genotypic characterization of an amylose-free starch mutant of the potato. Euphytica 44: 43-48
Kloosterman B, De Koeyer D, Griffiths R, Flinn B, Steuernagel B, Scholz U, Sonnewald S, Sonnewald U, Bryan GJ, Prat S, Bánfalvi Z, Hammond JP, Geigenberger P, Nielsen KL, Visser RGF, Bachem CWB (2008) Genes driving potato tuber initiation and growth: identification based on transcriptional changes using the POCI array. Funct Integr Genomics 8: 329-340
Kobayashi A, Ohara-Takada A, Tsuda S, Matsuura-Endo C, Takada N, Umemura Y, Nakao T, Yoshida T, Hayashi K, Mori M (2008) Breeding of potato variety "Inca-no-hitomi" with a very high carotenoid content. Breeding Science 58: 77-82
Kort J, Jaspers CP, Dijkstra DL (1972) Testing for resistance to pathotype C of Heterodera rostochiensis and the practical application of Solanum vernei-hybrids in the Netherlands. Annals of Applied Biology 71: 289-294
Krinsky NI, Mayne ST, Sies H (2004) Carotenoids in health and disease. CRC Press, New York, USA
Lightbourn GJ, Veilleux RE (2007) Production and evaluation of somatic hybrids derived from monoploid potato. Amer J Potato Res 84: 425-435
Lopez AB, Van Eck J, Conlin BJ, Paolillo DJ, O'Neill J, Li L (2008) Effect of the cauliflower Or transgene on carotenoid accumulation and chromoplast formation in transgenic potato tubers. J Exp Bot 59: 213-223
Maceira NO, De Haan AA, Lumaret R, Billon M, Delay J (1992) Production of 2n gametes in diploid subspecies of Dactylus glomerata L. 1. Occurrence and frequency of 2n pollen. Ann Bot 69: 335-343
Moeller SM, Parekh N, Tinker L, Ritenbaugh C, Blodi B, Wallace RB, Mares JA (2006) Associations between intermediate age-related macular degeneration and lutein and zeaxanthin in the carotenoids in age-related eye disease study (CAREDS). Arch Ophthalmol 124: 1151-1162
Morris WL, Ducreux L, Griffiths DW, Stewart D, Davies HV, Taylor MA (2004) Carotenogenesis during tuber development and storage in potato. J Exp Bot 55: 975-982
Nesterenko S, Sink KC (2003) Carotenoid profiles of potato breeding lines and selected cultivars. HortScience 38: 1173-1177
Ng PC, Henikoff S (2006) Predicting the effects of amino acid substitutions on protein function. Annu Rev Genomics Hum Genet 7: 61-80
Nicot N, Hausman JF, Hoffmann L, Evers D (2005) Housekeeping gene selection for real-time RT-PCR normalization in potato during biotic and abiotic stress. J Exp Bot 56: 2907-2914
Ohmori Y, Abiko M, Horibata A, Hirano HY (2008) A transposon, Ping, is integrated into intron 4 of the DROOPING LEAF gene of rice, weakly reducing its expression and causing a mild drooping leaf phenotype. Plant Cell Physiol 49: 1176-1184
Park TH, Vleeshouwers VGAA, Kim JB, Hutten RCB, Visser RGF (2005) Dissection of foliage and tuber late blight resistance in mapping populations of potato. Euphytica 143: 75-83
Ritter E, Barandalla L, López R, Ruiz de Galarreta JI (2008) Exploitation of exotic, cultivated Solanum germplasm for breeding and commercial purposes. Potato Res 51: 301-311
Rogers SO, Bendich AJ (1988) Extraction of DNA from plant tissues. In: Gelvin SB, Schilperoort RA (eds) Plant Molecular Biology Manual, pp. A6/1-A6/10. Kluwer Academic Publishers, Dordrecht, Netherlands
Römer S, Lübeck J, Kauder F, Steiger S, Adomat C, Sandmann G (2002) Genetic engineering of a zeaxanthin-rich potato by antisense inactivation and co-suppression of carotenoid epoxidation. Metabolic Engineering 4: 263-272
Rouppe van der Voort JNAM, van Zandvoort P, van Eck HJ, Folkertsma RT, Hutten RCB, Draaistra J, Gommers FJ, Jacobsen E, Helder J, Bakker J (1997) Use of allele specificity of comigrating AFLP markers to align genetic maps from different potato genotypes. Mol Gen Genet 255: 438-447
Sambrook, J, Russell DW, Sambrook, J (2001) Molecular Cloning: a Laboratory Manual. Cold Springs Harbor Laboratory Press, Plainview, N.Y.
Seddon JM, Ajani UA, Sperduto RD, Hiller R, Blair N, Burton TC, Farber MD, Gragoudas ES, Haller J, Miller DT et al. (1994) Dietary carotenoids, vitamins A, C, and E, and advanced age-related macular degeneration. Eye Disease Case-Control Study Group. JAMA 272: 1413-1420
Snodderly DM (1995) Evidence for protection against age-related macular degeneration by carotenoids and antioxidant vitamins. Am J Clin Nutr 62 (suppl): 1448S-1461S
Sommerburg O, Keunen JEE, Bird AC, van Kuijk FJGM (1998) Fruits and vegetables that are sources for lutein and zeaxanthin: the macular pigment in human eyes. Br J Ophthalmol 82: 907-910
Tai W, Dewey DR (1966) Morphology, cytology, and fertility of diploid and colchicine-induced tetraploid crested wheatgrass. Crop Sci 6: 223-226
Tanaka Y, Sasaki N, Ohmiya A (2008) Biosynthesis of plant pigments: anthocyanins, betalains and carotenoids. Plant J 54: 733-749
Thorup TA, Tanyolac B, Livingstone KD, Popovsky S, Paran I, Jahn M (2000) Candidate gene analysis of organ pigmentation loci in the Solanaceae. Proc Natl Acad Sci USA 97: 11192-11197
Uijtewaal BA (1987) Ploidy variability in greenhouse cultured and in vitro propagated potato (Solanum tuberosum) monohaploids (2n = x = 12) as determined by flow cytometry. Plant Cell Rep 6: 252-255
Uijtewaal BA, Huigen DJ, Hermsen JGTh (1987) Production of potato monohaploids (2n = x = 12) through prickle pollination. Theor Appl Genet 73: 751-758
Van der Beek JG, Verkerk R, Zabel P, Lindhout P (1992) Mapping stategy for resistance genes in tomato based on RFLPs between cultivars: CF9 (resistance to Cladosporium fulvum) on chromosome 1. Theor Appl Genet 84: 106-112
Van Ooijen JW (2004) MapQTL® 5. Software for the mapping of quantitative trait loci in experimental populations. Kyazma B.V., Wageningen, The Netherlands
Van Ooijen JW (2006) JoinMap® 4. Software for the calculation of genetic linkage maps in experimental populations. Kyazma B.V., Wageningen, The Netherlands
Van Os H, Andrzejewski S, Bakker E, Barrena I, Bryan GJ, Caromel B, Ghareeb B, Isidore E, de Jong W, van Koert P, Lefebvre V, Milbourne D, Ritter E, Rouppe van der Voort JNAM, Rousselle-Bourgeois F, van Vliet J, Waugh R, Visser RGF, Bakker J, van Eck HJ (2006) Construction of a 10,000-marker ultradense genetic recombination map of potato: providing a framework for accelerated gene isolation and a genomewide physical map. Genetics 173: 1075-1087.

## Claims

1. A method for increasing the level of zeaxanthin in a plant, comprising:
a) selecting at least one parental genotype comprising at least one recessive zeaxanthin epoxidase (ZEP) allele,
b) crossing said plant(s),
c) selecting progeny from said crossing for a genotype comprising at least one recessive ZEP allele,
d) selfing and /or further crossing said progeny selected in step a),
e) selecting progeny resulting from the crossing in step d) for a genotype comprising at least one recessive ZEP allele,
f) optionally repeating said steps of selfing and/or crossing and selection of steps d) and e) to provide a plant having a genotype that is homozygous or quadruplex for said recessive ZEP allele,
wherein at least one selection as performed in steps a), c) or e) is performed by marker-assisted selection and wherein said method optionally comprises increasing the ploidy or the use of unreduced gametes of the parental genotype selected at step a) or the progeny of step c) to tetraploid level before conducting step e).

2. The method according to claim 1, wherein the genotype of the plant in step f) comprises at least one dominant CHY2 allele, preferably wherein said CHY2 allele is indicated by a marker, selected from the group of the CAPS marker based on the PCR product generated with primers CHY2ex4F and Beta-R822 (Table 1, Figure 2), any SNP selected from Table 2detectable within PCR amplicon product CHY2ex4F and Beta-R822, for example SNP T142C, and primer sequence yellowF1.

3. A method for selecting a plant or part thereof, including a seed and tuber, said method comprising:
a) testing a plant or part thereof for the presence of at least one marker that is indicative for a recessive ZEP allele, and
b) selecting said plant or part thereof based on the information derived from said testing; and
c) optionally further testing a plant or part thereof for the presence of at least one marker that is indicative for a dominant CHY2 allele, and selecting said plant or part thereof based on the information derived from said testing.

4. The method according to any one of claims 1 to 3, wherein said recessive ZEP allele comprises the nucleic acid sequence of ZEP allele 1 in Figure 12.

5. The method according to any one of claims 1 to 4, wherein the presence of said marker is determined at DNA level.

6. The method according to any one of claims 1 to 5, wherein said marker is selected from the group consisting of a single nucleotide polymorphism (SNP), an insertion and an indel, preferably wherein said insertion is a transposon-like insertion, more preferably wherein said transposon-like insertion is the transposon-like insertion in intron 1 of the nucleic acid sequence of ZEP allele 1 in Figure 12.

7. The method according to claim 6, wherein said indel is the 49-base pairs indel in exon 4 of the nucleic acid sequence of ZEP allele 1 in Figure 12.

8. The method according to any one of claims 1 to 7, wherein said plant is tetraploid.

9. The method according to any one of claims 1 to 8, wherein said plant is a potato plant, preferably wherein said potato plant is a *S*. *tuberosum* Group Tuberosum cultivar.

10. Use of a marker for selecting a plant or part thereof, including a seed and tuber, with a genotype comprising at least one recessive ZEP allele, wherein said marker is a SNP selected from the group consisting of G83A, T99G, A113T, A154G, G165C, A180G, T209C, T231A, T375G, A384T, A389C, A415G, C422-, C422T, T426- and T426C, the transposon insertion in intron 1 of the nucleic acid sequence of ZEP allele 1 in Figure 12 or the 49-base pairs indel in exon 4 of the nucleic acid sequence of ZEP allele 1 in Figure 12.

11. The use according to claim 10, wherein said ZEP allele comprises the nucleic acid sequence of ZEP allele 1 in Figure 12.

12. Use of a method according to claim 3 for marker-assisted crossing of a plant.

13. The use according to any one of claims 10 to 12, wherein said plant is tetraploid.

14. The use according to any one of claims 10 to 13, wherein said plant is a potato plant, preferably wherein said potato plant is a *S*. *tuberosum* Group Tuberosum cultivar.

15. A probe for detecting a marker in a plant or part thereof, including a seed and tuber, wherein said marker is indicative for a ZEP allele comprising the nucleic acid sequence of ZEP allele 1 in Figure 12, preferably wherein said marker is a SNP selected from the group consisting of G83A, T99G, A113T, A154G, G165C, A180G, T209C, T231A, T375G, A384T, A389C, A415G, C422-, C422T, T426- and T426C, the transposon insertion in intron 1 of the nucleic acid sequence of ZEP allele 1 in Figure 12 or the 49-base pairs indel in exon 4 of the nucleic acid sequence of ZEP allele 1 in Figure 12.

16. Use of a probe according to claim 15 for marker-assisted breeding of a plant, preferably wherein said plant is tetraploid, more preferably wherein said plant is a potato plant, more preferably wherein said potato plant is a *S*. *tuberosum* Group *tuberosum* cultivar.

17. A tetraploid potato plant having tubers with an increased zeaxanthin content, wherein the genotype of said potato comprises homozygous recessive ZEP alleles comprising the nucleic acid sequence of ZEP allele 1 in Figure 12, preferably wherein said plant comprises at least one dominant CHY2 allele.

18. The potato plant according to claim 17, wherein said potato is a *S*. *tuberosum* Group Tuberosum cultivar, preferably wherein said potato plant is a tetraploid *S*. *tuberosum* Group Tuberosum cultivar.

19. A tuber with deep yellow or orange flesh produced by a plant according to claim 17 or 18.

20. A food product comprising a tuber according to claim 19.
